# EUROPEAN PATENT SPECIFICATION

(11) **EP 0 625 159 B1**
(45) Date of publication and mention of the grant of the patent: **26.03.2003**
(21) Application number: 93921618.0
(22) Date of filing: 14.09.1993
(51) Int. Cl.: C12N 15/12, C07K 14/47, C12N 15/63, C12N 5/10, C07K 16/18, C12P 21/08, A61K 38/17, A61K 48/00, C12Q 1/68, A01K 67/027

(54) **IKAROS: A REGULATORY GENE INVOLVED IN T-CELL DIFFERENTIATION**
IKAROS: EIN REGULATORISCHES GEN BEI DER T-ZELL DIFFERENZIERUNG
IKAROS: GENE REGULATEUR DU PROCESSUS DE DIFFERENTIATION DES LYMPHOCYTES T

(30) Priority: 14.09.1992 US 946233
(43) Date of publication of application: 23.11.1994
(73) Proprietor: THE GENERAL HOSPITAL CORPORATION, Boston, MA 02114 (US)
(72) Inventor: GEORGOPOULOS, Katia, Cambridge, MA 02138 (US)
(74) Representative: Ruffles, Graham Keith
(86) International application number: US9308743
(87) International publication number: WO94006814

(56) References cited:
- EMBO JOURNAL, vol. 9, no. 1, 1990, pages 109-115, XP002022648 GEORGOPOULOS ET AL. : "Tissue-specific nuclear factors mediate expression of the CD3-delta gene during T cell development."
- CELL, vol. 52, 12 February 1988, pages 415-423, XP002022649 SINGH ET AL.: "Molecular cloning of an enhancer binding protein: Isolation by screening of an expression library with a recognition site DNA."
- SCIENCE, vol. 258, 30 October 1992, pages 808-812, XP002022650 GEORGOPOULOS K. ET AL.: "Ikaros, an early lymphoid-specific transcription factor and a putative mediator for T cell commitment."
- JOURNAL OF CELLULAR BIOCHEMISTRY, vol. SUPPL.17A, January 1993, page B631 XP000615962 GEORGOPOULOS K. : "Ikaros an early lymphoid restricted regulatory protein, a putative modulator for T cell specification."
- No relevant art found.

### SEQUENCE LISTING

(1) GENERAL INFORMATION:
   (i) APPLICANT:
      (A) NAME: The General Hospital Corporation
      (B) STREET: 55 Fruit Street
      (C) CITY: Boston
      (D) STATE: Massachusetts
      (E) COUNTRY: USA
      (F) POSTAL CODE (ZIP): 02114
      (G) TELEPHONE: (617) 726-8608
      (H) TELEFAX: (617) 726-1668
   (ii) TITLE OF INVENTION: IKAROS: A T CELL PATHWAY REGULATORY GENE
   (iii) NUMBER OF SEQUENCES: 152
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII
   (v) CURRENT APPLICATION DATA:
      (A) APPLICATION NUMBER:
   (vi) PRIOR APPLICATION DATA:
      (A) APPLICATION NUMBER: US 946,233
      (B) FILING DATE: 14-SEP-1992
   (ix) TELECOMMUNICATION INFORMATION:
      (A) TELEPHONE: (617)227-7400
      (B) TELEFAX: (617)227-5941
(2) INFORMATION FOR SEQ ID NO:1:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDHA
   (xi) SEQUENCE DESCRIPTION: SEQ ID NO:1:
   AGAAGTTTCC ATGACATCAT GAATGGGGGT GGCAGAGA 38
(2) INFORMATION FOR SEQ ID NO:2:
   (i) SEQUENCE CHARACTERISTICS:
      (A) LENGTH: 1788 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 223..1515
   (xi) SEQUENCE DESCRIPTION: SEQ ID NO:2:
   AATTCGTTCT ACCTTCTCTG AACCCCAGTG GTGTGTCAAG GCCGGACTGG GAGCTTGGGG   60
   GAAGAGGAAG AGGAAGAGGA ATCTGCGGCT CATCCAGGGA TCAGGGTCCT TCCCAAGTGG  120
   CCACTCAGAG GGGACTCAGA GCAAGTCTAG ATTTGTGTGG CAGAGAGAGA CAGCTCTCGT  180
   TTGGCCTTGG GGAGGCACAA GTCTGTTGAT AACCTGAAGA CA                     222
   ATG GAT GTC GAT GAG GGT CAA GAC ATG TCC CAA GTT TCA GGA AAG GAG    270
   Met Asp Val Asp Glu Gly Gln Asp Met Ser Gln Val Ser Gly Lys Glu
   1               5                   10                  15
   AGC CCC CCA GTC AGT GAC ACT CCA GAT GAA GGG GAT GAG CCC ATG CCT    318
   Ser Pro Pro Val Ser Asp Thr Pro Asp Glu Gly Asp Glu Pro Met Pro
               20                  25                  30
   GTC CCT GAG GAC CTG TCC ACT ACC TCT GGA GCA CAG CAG AAC TCC AAG    366
   Val Pro Glu Asp Leu Ser Thr Thr Ser Gly Ala Gln Gln Asn Ser Lys
           35                  40                  45
   AGT GAT CGA GGC ATG GGT GAA CGG CCT TTC CAG TGC AAC CAG TCT GGG    414
   Ser Asp Arg Gly Met Gly Gln Arg Pro Phe Gln Cys Asn Gln Ser Gly
       50                  55                  60
   GCC TCC TTT ACC CAG AAA GGC AAC CTC CTG CGG CAC ATC AAG CTG CAC    462
   Ala Ser Phe Thr Gln Lys Gly Asn Leu Leu Arg His Ile Lys Leu His
   65                  70                  75                  80
   TCG GGT GAG AAG CCC TTC AAA TGC CAT CTT TGC AAC TAT GCC TGC CGC    510
   Ser Gly Glu Lys Pro Phe Lys Cys His Leu Cys Asn Tyr Ala Cys Arg
                   85                  90                  95
   CGG AGG GAC GCC CTC ACC GGC CAC CTG AGG ACG CAC TCC GTT GGT AAG     558
   Arg Arg Asp Ala Leu Thr Gly His Leu Arg Thr His Ser Val Gly Lys
               100                 105                 110
   CCT CAC AAA TGT GGA TAT TGT GGC CGG AGC TAT AAA CAG CGA AGC TCT     606
   Pro His Lys Cys Gly Tyr Cys Gly Arg Ser Tyr Lys Gln Arg Ser Ser
           115                 120                 125
   TTA GAG GAG CAT AAA GAG CGA TGC CAC AAC TAC TTG GAA AGC ATG GGC     654
   Leu Glu Glu His Lys Glu Arg Cys His Asn Tyr Leu Glu Ser Met Gly
       130                 135                 140
   CTT CCG GGC GTG TGC CCA GTC ATT AAG GAA GAA ACT AAC CAC AAC GAG     702
   Leu Pro Gly Val Cys Pro Val Ile Lys Glu Glu Thr Asn His Asn Glu
   145                 150                 155                 160
   ATG GCA GAA GAC CTG TGC AAG ATA GGA GCA GAG AGG TCC CTT GTC CTG     750
   Met Ala Glu Asp Leu Cys Lys Ile Gly Ala Glu Arg Ser Leu Val Leu
                   165                 170                 175
   GAC AGG CTG GCA AGC AAT GTC GCC AAA CGT AAG AGC TCT ATG CCT CAG     798
   Asp Arg Leu Ala Ser Asn Val Ala Lys Arg Lys Ser Ser Met Pro Gln
               180                 185                 190
   AAA TTT CTT GGA GAC AAG TGC CTG TCA GAC ATG CCC TAT GAC AGT GCC     846
   Lys Phe Leu Gly Asp Lys Cys Leu Ser Asp Met Pro Tyr Asp Ser Ala
           195                 200                 205
   AAC TAT GAG AAG GAG GAT ATG ATG ACA TCC CAC GTG ATG GAC CAG GCC     894
   Asn Tyr Glu Lys Glu Asp Met Met Thr Ser His Val Met Asp Gln Ala
       210                 215                 220
   ATC AAC AAT GCC ATC AAC TAC CTG GGG GCT GAG TCC CTG CGC CCA TTG     942
   Ile Asn Asn Ala Ile Asn Tyr Leu Gly Ala Glu Ser Leu Arg Pro Leu
   225                 230                 235                 240
   GTG CAG ACA CCC CCC GGT AGC TCC GAG GTG GTG CCA GTC ATC AGC TCC     990
   Val Gln Thr Pro Pro Gly Ser Ser Glu Val Val Pro Val Ile Ser Ser
                   245                 250                 255
   ATG TAC CAG CTG CAC AAG CCC CCC TCA GAT GGC CCC CCA CGG TCC AAC    1038
   Met Tyr Gln Leu His Lys Pro Pro Ser Asp Gly Pro Pro Arg Ser Asn
               260                 265                 270
   CAT TCA GCA CAG GAC GCC GTG GAT AAC TTG CTG CTG CTG TCC AAG GCC    1086
   His Ser Ala Gln Asp Ala Val Asp Asn Leu Leu Leu Leu Ser Lys Ala
           275                 280                 285
   AAG TCT GTG TCA TCG GAG CGA GAG GCC TCC CCG AGC AAC AGC TGC CAA    1134
   Lys Ser Val Ser Ser Glu Arg Glu Ala Ser Pro Ser Asn Ser Cys Gln
       290                 295                 300
   GAC TCC ACA GAT ACA GAG AGC AAC GCG GAG GAA CAG CGC AGC GGC CTT    1182
   Asp Ser Thr Asp Thr Glu Ser Asn Ala Glu Glu Gln Arg Ser Gly Leu
   305                 310                 315                 320
   ATC TAC CTA ACC AAC CAC ATC AAC CCG CAT GCA CGC AAT GGG CTG GCT    1230
   Ile Tyr Leu Thr Asn His Ile Asn Pro His Ala Arg Asn Gly Leu Ala
                   325                 330                 335
   CTC AAG GAG GAG CAG CGC GCC TAC GAG GTG CTG AGG GCG GCC TCA GAG    1278
   Leu Lys Glu Glu Gln Arg Ala Tyr Glu Val Leu Arg Ala Ala Ser Glu
               340                 345                 350
   AAC TCG CAG GAT GCC TTC CGT GTG GTC AGC ACG AGT GGC GAG CAG CTG    1326
   Asn Ser Gln Asp Ala Phe Arg Val Val Ser Thr Ser Gly Glu Gln Leu
           355                 360                 365
   AAG GTG TAC AAG TGC GAA CAC TGC CGC GTG CTC TTC CTG GAT CAC GTC    1374
   Lys Val Tyr Lys Cys Glu His Cys Arg Val Leu Phe Leu Asp His Val
       370                 375                 380
   ATG TAT ACC ATT CAC ATG GGC TGC CAT GGC TGC CAT GGC TTT CGG GAT    1422
   Met Tyr Thr Ile His Met Gly Cys His Gly Cys His Gly Phe Arg Asp
   385                 390                 395                 400
   CCC TTT GAG TGT AAC ATG TGT GGT TAT CAC AGC CAG GAC AGG TAC GAG    1470
   Pro Phe Glu Cys Asn Met Cys Gly Tyr His Ser Gln Asp Arg Tyr Glu
                   405                 410                 415
   TTC TCA TCC CAT ATC ACG CGG GGG GAG CAT CGT TAC CAC CTG AGC        1515
   Phe Ser Ser His Ile Thr Arg Gly Glu His Arg Tyr His Leu Ser
               420                 425                 430
   TAAACCCAGC CAGGCCCCAC TGAAGCACAA AGATAGCTGG TTATGCCTCC TTCCCGGCAG  1575
   CTGGACCCAC AGCGGACAAT GTGGGAGTGG ATTTGCAGGC AGCATTTGTT CTTTTATGTT  1635
   GGTTGTTTGG CGTTTCATTT GCGTTGGAAG ATAAGTTTTT AATGTTAGTG ACAGGATTGC  1695
   ATTGCATCAG CAACATTCAC AACATCCATC CTTCTAGCCA GTTTTGTTCA CTGGTAGCTG  1755
   AGGTTTCCCG GATATGTGGC TTCCTAACAC TCT                               1788
(2) INFORMATION FOR SEQ ID NO:3:
   (i) SEQUENCE-CHARACTERISTICS:
      (A) LENGTH: 1611 base pairs
      (B) TYPE: nucleic acid
      (C) STRAMDEDNESS: single
      (D) TOPOLOGY: linear
   (ii) MOLECULE TYPE: cDNA
   (ix) FEATURE:
      (A) NAME/KEY: CDS
      (B) LOCATION: 1..1611
   (xi) SEQUENCE DESCRIPTION: SEQ ID NO:3:
   GAA TTC CGC CTT TGG GGG CTG ACC GGG AGC CCG GCG CGA TTG CAA AGT     48
   Glu Phe Arg Leu Trp Gly Leu Thr Gly Ser Pro Ala Arg Leu Gln Ser
     1               5                  10                  15
   TTT CGT GCG CGC CCC TCT GGC CCG GAG TTG CGG CTG AGA CGC GCC GCG     96
   Phe Arg Ala Arg Pro Ser Gly Pro Glu Leu Arg Leu Arg Arg Ala Ala
                    20                  25                  30
   CGA GCC GGG GGA CTC GGC GAC GGG GCG GGG ACG GGA CGA CGC ACC CTC    144
   Arg Ala Gly Gly Leu Gly Asp Gly Ala Gly Thr Gly Arg Arg Thr Leu
            35                  40                  45
   TCC GTG TCC GCT CTC GCC CTT CTG CGC GCC CCG CTC CCT GTA CCG GAG    192
   Ser Val Ser Ala Leu Ala Leu Leu Arg Ala Pro Leu Pro Val Pro Glu
        50                  55                  60
   CAG CGA TCC GGG AGG CGG CCG AGA GCC AGT AAT GTT AAA GTA GAG ACT    240
   Gln Arg Ser Gly Arg Arg Pro Arg Ala Ser Asn Val Lys Val Glu Thr
    65                  70                  75                  80
   CAG AGT GAT GAA GAG AAT GGG CGT GCC TGT GAA ATG AAT GGG GAA GAA    288
   Gln Ser Asp Glu Glu Asn Gly Arg Ala Cys Glu Met Asn Gly Glu Glu
                    85                  90                  95
   TGT GCG GAG GAT TTA CGA ATG CTT GAT GCC TCG GGA GAG AAA ATG AAT    336
   Cys Ala Glu Asp Leu Arg Met Leu Asp Ala Ser Gly Glu Lys Met Asn
               100                 105                 110
   GGC TCC CAC AGG GAC CAA GGC AGC TCG GCT TTG TCG GGA GTT GGA GGC    384
   Gly Ser His Arg Asp Gln Gly Ser Ser Ala Leu Ser Gly Val Gly Gly
           115                 120                 125
   ATT CGA CTT CCT AAC GGA AAA CTA AAG TGT GAT ATC TGT GGG ATC ATT    432
   Ile Arg Leu Pro Asn Gly Lys Leu Lys Cys Asp Ile Cys Gly Ile Ile
       130                 135                 140
   TGC ATC GGG CCC AAT GTG CTC ATG GTT CAC AAA AGA AGC CAC ACT GGA    480
   Cys Ile Gly Pro Asn Val Leu Met Val His Lys Arg Ser His Thr Gly
   145                 150                 155                 160
   GAA CGG CCC TTC CAG TGC AAT CAG TGC GGG GCC TCA TTC ACC CAG AAG    528
   Glu Arg Pro Phe Gln Cys Asn Gln Cys Gly Ala Ser Phe Thr Gln Lys
                   165                 170                 175
   GGC AAC CTG CTC CGG CAC ATC AAG CTG CAT TCC GGG GAG AAG CCC TTC    576
   Gly Asn Leu Leu Arg His Ile Lys Leu His Ser Gly Glu Lys Pro Phe
               180                 185                 190
   AAA TGC CAC CTC TGC AAC TAC GCC TGC CGC CGG AGG GAC GCC CTC ACT    624
   Lys Cys His Leu Cys Asn Tyr Ala Cys Arg Arg Arg Asp Ala Leu Thr
           195                 200                 205
   GGC CAC CTG AGG ACG CAC TCC GTT GGT AAA CCT CAC AAA TGT GGA TAT    672
   Gly His Leu Arg Thr His Ser Val Gly Lys Pro His Lys Cys Gly Tyr
       210                 215                 220
   TGT GGC CGA AGC TAT AAA CAG CGA ACG TCT TTA GAG GAA CAT AAA GAG 720
   Cys Gly Arg Ser Tyr Lys Gln Arg Thr Ser Leu Glu Glu His Lys Glu
   225 230 235 240
   CGC TGC CAC AAC TAC TTG GAA AGC ATG GGC CTT CCG GGC ACA CTG TAC 768
   Arg Cys His Asn Tyr Leu Glu Ser Met Gly Leu Pro Gly Thr Leu Tyr
   245 250 255
   CCA GTC ATT AAA GAA GAA ACT AAG CAC AGT GAA ATG GCA GAA GAC CTG 816
   Pro Val Ile Lys Glu Glu Thr Lys His Ser Glu Met Ala Glu Asp Leu
   260 265 270
   TGC AAG ATA GGA TCA GAG AGA TCT CTC GTG CTG GAC AGA CTA GCA AGT 864
   Cys Lys Ile Gly Ser Glu Arg Ser Leu Val Leu Asp Arg Leu Ala Ser
   275 280 285
   AAT GTC GCC AAA CGT AAG AGC TCT ATG CCT CAG AAA TTT CTT GGG GAC 912
   Asn Val Ala Lys Arg Lys Ser Ser Met Pro Gln Lys Phe Leu Gly Asp
   290 295 300
   AAG GGC CTG TCC GAC ACG CCC TAC GAC AGT GCC ACG TAC GAG AAG GAG 960
   Lys Gly Leu Ser Asp Thr Pro Tyr Asp Ser Ala Thr Tyr Glu Lys Glu
   305 310 315 320
   AAC GAA ATG ATG AAG TCC CAC GTG ATG GAC CAA GCC ATC AAC AAC GCC 1008
   Asn Glu Met Met Lys Ser His Val Met Asp Gln Ala Ile Asn Asn Ala
   325 330 335
   ATC AAC TAC CTG GGG GCC GAG TCC CTG CGC CCG CTG GTG CAG ACG CCC 1056
   Ile Asn Tyr Leu Gly Ala Glu Ser Leu Arg Pro Leu Val Gln Thr Pro
   340 345 350
   CCG GGC GGT TCC GAG GTG GTC CCG GTC ATC AGC CCG ATG TAC CAG CTG 1104
   Pro Gly Gly Ser Glu Val Val Pro Val Ile Ser Pro Met Tyr Gln Leu
   355 360 365
   CAC AGG CGC TCG GAG GGC ACC CCG CGC TCC AAC CAC TCG GCC CAG GAC 1152
   His Arg Arg Ser Glu Gly Thr Pro Arg Ser Asn His Ser Ala Gln Asp
   370 375 380
   AGC GCC GTG GAG TAC CTG CTG CTG CTC TCC AAG GCC AAG TTG GTG CCC 1200
   Ser Ala Val Glu Tyr Leu Leu Leu Leu Ser Lys Ala Lys Leu Val Pro
   385 390 395 400
   TCG GAG CGC GAG GCG TCC CCG AGC AAC AGC TGC CAA GAC TCC ACG GAC 1248
   Ser Glu Arg Glu Ala Ser Pro Ser Asn Ser Cys Gln Asp Ser Thr Asp
   405 410 415
   ACC GAG AGC AAC AAC GAG GAG CAG CGC AGC GGT CTT ATC TAC CTG ACC 1296
   Thr Glu Ser Asn Asn Glu Glu Gln Arg Ser Gly Leu Ile Tyr Leu Thr
   420 425 430
   AAC CAC ATC GCC CGA CGC GCG CAA CGC GTG TCG CTC AAG GAG GAG CAC 1344
   Asn His Ile Ala Arg Arg Ala Gln Arg Val Ser Leu Lys Glu Glu His
   435 440 445
   CGC GCC TAC GAC CTC GTG CGC GCC GCC TCC GAG AAC TCG CAG GAC GCG     1392
   Arg Ala Tyr Asp Leu Val Arg Ala Ala Ser Glu Asn Ser Gln Asp Ala
       450                 455                 460
   TTC CGC GTG GTC AGC ACC AGC GGG GAG CAG ATG AAG GTG TAC AAG TGC     1440
   Phe Arg Val Val Ser Thr Ser Gly Glu Gln Met Lys Val Tyr Lys Cys
   465                 470                 475                 480
   GAA CAC TGC CGG GTG CTC TTC CTG GAT CAC GTC ATG TAC ACC ATC CAC     1488
   Glu His Cys Arg Val Leu Phe Leu Asp His Val Met Tyr Thr Ile His
                   485                 490                 495
   ATG GGC TGC CAC GGC TTC CGT GAT CCT TTT GAG TGC ACC ATG TGC GGC     1536
   Met Gly Cys His Gly Phe Arg Asp Pro Phe Glu Cys Thr Met Cys Gly
               500                 505                 510
   TAC CAC AGC CAG GAC CGG TAC GAG TTC TCG TCG CAC ATA ACG CGA GGG     1584
   Tyr His Ser Gln Asp Arg Tyr Glu Phe Ser Ser His Ile Thr Arg Gly
           515                 520                 525
   GAG CAC CGC TTC CAC ATG ACG TAA GCC                                 1611
   Glu His Arg Phe His Met Thr  *  Ala
       530                 535
(2) INFORMATION FOR SEQ ID NO:4:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   AGAAGTTTCC ATAAGATGAT GAATGGGGGT GGCAGAGA     38
(2) INFORMATION FOR SEQ ID NO:5:
   (i) SEQUENCE CHARACTERISTICS:
      (A) LENGTH: 568 amino acids
      (B) TYPE: amino acid
      (C) STRANDEDNESS: single
      (D) TOPOLOGY: linear
   (ii) MOLECULE TYPE: peptide
   (v) FRAGMENT TYPE: internal
   (xi) SEQUENCE DESCRIPTION: SEQ ID NO:5:
   Met Asp Val Asp Glu Gly Gln Asp Met Ser Gln Val Ser Gly Lys Glu
   1 5 10 15
   Ser Pro Pro Val Ser Asp Thr Pro Asp Glu Gly Asp Glu Pro Met Pro
   20 25 30
   Val Pro Glu Asp Leu Ser Thr Thr Ser Gly Ala Gln Gln Asn Ser Lys
   35 40 45
   Ser Asp Arg Gly Met Ala Ser Asn Val Lys Val Glu Thr Gln Ser Asp
   50 55 60
   Glu Glu Asn Gly Arg Ala Cys Glu Met Asn Gly Glu Glu Cys Ala Glu
   65 70 75 80
   Asp Leu Arg Met Leu Asp Ala Ser Gly Glu Lys Met Asn Gly Ser His
   85 90 95
   Arg Asp Gln Gly Ser Ser Ala Leu Ser Gly Val Gly Gly Ile Arg Leu
   100 105 110
   Pro Asn Gly Lys Leu Lys Cys Asp Ile Cys Gly Ile Val Cys Ile Gly
   115 120 125
   Pro Asn Val Leu Met Val His Lys Arg Ser His Thr Gly Glu Arg Pro
   130 135 140
   Phe Gln Cys Asn Gln Cys Ser Ser Ala Leu Ser Gly Val Gly Gly Ile
   145 150 155 160
   Arg Leu Pro Asn Gly Lys Leu Lys Cys Asp Ile Cys Gly Ile Val Cys
   165 170 175
   Ile Gly Pro Asn Val Leu Met Val His Lys Arg Ser His Thr Gly Glu
   180 185 190
   Arg Pro Phe Gln Cys Asn Gln Cys Gly Ala Ser Phe Thr Gln Lys Gly
   195 200 205
   Asn Leu Leu Arg His Ile Lys Leu His Ser Gly Glu Lys Pro Phe Lys
   210 215 220
   Cys His Leu Cys Asn Tyr Ala Cys Arg Arg Arg Asp Ala Leu Thr Gly
   225 230 235 240
   His Leu Arg Thr His Ser Val Gly Lys Pro His Lys Cys Gly Tyr Cys
   245 250 255
   Gly Arg Ser Tyr Lys Gln Arg Ser Ser Leu Glu Glu His Lys Glu Arg
   260 265 270
   Cys His Asn Tyr Leu Glu Ser Met Gly Leu Pro Gly Met Tyr Pro Val
   275 280 285
   Ile Lys Glu Glu Thr Asn His Asn Glu Met Ala Glu Asp Leu Cys Lys
   290 295 300
   Ile Gly Ala Glu Arg Ser Leu Val Leu Asp Arg Leu Ala Ser Asn Val
   305                 310                 315                 320
   Ala Lys Arg Lys Ser Ser Met Pro Gln Lys Phe Leu Gly Asp Lys Cys
                   325                 330                 335
   Leu Ser Asp Met Pro Tyr Asp Ser Ala Asn Tyr Glu Lys Glu Asp Met
               340                 345                 350
   Met Thr Ser His Val Met Asp Gln Ala Ile Asn Asn Ala Ile Asn Tyr
           355                 360                 365
   Leu Gly Ala Glu Ser Leu Arg Pro Leu Val Gln Thr Pro Pro Gly Ser
       370                 375                 380
   Ser Glu Val Val Pro Val Ile Ser Ser Met Tyr Gln Leu His Lys Pro
   385                 390                 395                 400
   Pro Ser Asp Gly Pro Pro Arg Ser Asn His Ser Ala Gln Asp Ala Val
                   405                 410                 415
   Asp Asn Leu Leu Leu Leu Ser Lys Ala Lys Ser Val Ser Ser Glu Arg
               420                 425                 430
   Glu Ala Ser Pro Ser Asn Ser Cys Gln Asp Ser Thr Asp Thr Glu Ser
           435                 440                 445
   Asn Ala Glu Glu Gln Arg Ser Gly Leu Ile Tyr Leu Thr Asn His Ile
       450                 455                 460
   Asn Pro His Ala Arg Asn Gly Leu Ala Leu Lys Glu Glu Gln Arg Ala
   465                 470                 475                 480
   Tyr Glu Val Leu Arg Ala Ala Ser Glu Asn Ser Gln Asp Ala Phe Arg
                   485                 490                 495
   Val Val Ser Thr Ser Gly Glu Gln Leu Lys Val Tyr Lys Cys Glu His
               500                 505                 510
   Cys Arg Val Leu Phe Leu Asp His Val Met Tyr Thr Ile His Met Gly
           515                 520                 525
   Cys His Gly Cys His Gly Phe Arg Asp Pro Phe Glu Cys Asn Met Cys
       530                 535                 540
   Gly Tyr His Ser Gln Asp Arg Tyr Glu Phe Ser Ser His Ile Thr Arg
   545                 550                 555                 560
   Gly Glu His Arg Tyr His Leu Ser
                   565
(2) INFORMATION FOR SEQ ID NO:6:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
   HNHTGGGAAT DYY 13
(2) INFORMATION FOR SEQ ID NO:7:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
   NNYYGGGAAT HNC 13
(2) INFORMATION FOR SEQ ID NO:8:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDHA
   (xi) SEQUENCE DESCRIPTION: SEQ ID NO:8:
   TMYGGGAATD YY 12
(2) INFORMATION FOR SEQ ID NO:9:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   AGGCGATTTT GGGAATTTCA CACC 24
(2) INFORMATION FOR SEQ ID NO:10:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:10:
   AGGCCATGGG AATGAAGGAA CACC 24
(2) INFORMATION FOR SEQ ID NO:11:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   GGTGTAAATT GGGAATGCTG TGCCT 25
(2) INFORMATION FOR SEQ ID NO:12:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   AGGCATGGGA ATGTCTGGAA CACC 24
(2) INFORMATION FOR SEQ ID NO:13:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
   AGGCATTAAA ATGGGAATAA CACC 24
(2) INFORMATION FOR SEQ ID NO:14:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   GGTGTAGGAA TGCGGTAATT GCCT 24
(2) INFORMATION FOR SEQ ID NO:15:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   GGTGTGGGAA TAACTGGGAT GCCT 24
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   GGTGTGGGAA TGTCACTTCA GCCT 24
(2) INFORMATION FOR SEQ ID NO:17:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
   GGTGTGGGAA TACTGAGTAT GCCTGCCT 28
(2) INFORMATION FOR SEQ ID NO:18:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
   AGGCAAATTT GGGAATACTA CACC 24
(2) INFORMATION FOR SEQ ID NO:19:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
   GGTGTGTGGG AACATGGGAT GCCT 24
(2) INFORMATION FOR SEQ ID NO:20:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: CDNA
   (xi) SEQUENCE DESCRIPTION: SEQ ID NO:20:
   AGGCCTATTT CCCTTGGGAA CACC 24
(2) INFORMATION FOR SEQ ID NO:21:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   GGTGTGGAAC ATCGTGGGAA GCCGCCT 27
(2) INFORMATION FOR SEQ ID NO:22:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
   AGGCGCTTGG GAAATTCCAA CACC 24
(2) INFORMATION FOR SEQ -ID NO:23:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
(2) INFORMATION FOR SEQ ID NO:24:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
   AGGCACAATT CCTTCGGGAA CACC 24
(2) INFORMATION FOR SEQ ID NO:25:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:25:
   GGTGTCGGGC TTCGGGAATA GCCT 24
(2) INFORMATION FOR SEQ ID NO:26:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:26:
   GGTGTTCCAA ACTCGGGAAT GCCT 24
(2) INFORMATION FOR SEQ ID NO:27:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:27:
   GGTGTGGAAT CGGGAATTTA GCCT 24
(2) INFORMATION FOR SEQ ID NO:28:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:28:
   AGGCTTATCG GGAAAACTTA CACC 24
(2) INFORMATION FOR SEQ ID NO:29:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:29:
   GGTGTTCCAA ACGGGGGAAT GCCT 24
(2) INFORMATION FOR SEQ ID NO:30:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:30:
   GGTGTGCAAT TCCAAGGAAT GCCT 24
(2) INFORMATION FOR SEQ ID NO:31:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:31:
   AGGCGCCATT CCAAGGATAA CACC 24
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:32:
   AGGCTAATCT TGGAATTCCA CACC 24
(2) INFORMATION FOR SEQ ID NO:33:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:33:
   GTGTGGACCA TTGGGATGCG CCT 23
(2) INFORMATION FOR SEQ ID NO:34:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:34:
   GGTGTTCCAA GAATCAGGAT GCCT 24
(2) INFORMATION FOR SEQ ID NO:35:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:35:
   ANTTGGGAAT RYY 13
(2) INFORMATION FOR SEQ ID NO:36:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:36:
   GGTGTACGGT TGGGAATGCG GCCT 24
(2) INFORMATION FOR SEQ ID NO:37
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:37:
   GGTGTAGGAA TGGGAATACA GCCT 24
(2) INFORMATION FOR SEQ ID NO:38:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:38:
   GGTGTTGGGA TTGGGAATGT GCCT 24
(2) INFORMATION FOR SEQ ID NO:39:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:39:
   GGTGTCGGGA ATTATTTTAG GCCT 24
(2) INFORMATION FOR SEQ ID NO:40:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:40:
   GGTGTAAAAA TGGGAACAAA GCCT 24
(2) INFORMATION FOR SEQ ID NO:41:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:41:
   GGTGTGGGAA AGATATAGCC GCCT 24
(2) INFORMATION FOR SEQ ID NO:42:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:42:
   GGTGTTTAAC CAATTGGGAA GCCT 24
(2) INFORMATION FOR SEQ ID NO:43:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:43:
   GGTGTTCCGG TATTTGGGAA GCCT 24
(2) INFORMATION FOR SEQ ID NO:44:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:44:
   GGTGTGGGAT AACTTGGGAA GCCT 24
(2) INFORMATION FOR SEQ ID NO:45:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:45:
   AGGCGGGAAA ACCCATAGGA CACC 24
(2) INFORMATION FOR SEQ ID NO:46:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:46:
   GGTGTAATCC GTCGGGAACA GCCTA 25
(2) INFORMATION FOR SEQ ID NO:47:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:47:
   GGCTTTAGAT CAGGGAACAC ACC 23
(2) INFORMATION FOR SEQ ID NO:48:
   (i) SEQUENCE CHARACTERISTICS:
      (A) LENGTH: 24 base pairs
      (B) TYPE: nucleic acid
      (C) STRAHDEDNESS: single
      (D) TOPOLOGY: linear
   (ii) MOLECULE TYPE: cDNA
   (xi) SEQUENCE DESCRIPTION: SEQ ID NO:48:
   GGTGTATCCT GGTAGGAATC GCCT 24
(2) INFORMATION FOR SEQ ID NO:49:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:49:
   AGGCTATCCC AGGAATTTGA CACC 24
(2) INFORMATION FOR SEQ ID NO:50:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:50:
   AGGCAAATTG TTCAGGAACA CACACC 26
(2) INFORMATION FOR SEQ ID NO:51:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:51:
   GGTGTCCATA AGGAACAATA GCCT 24
(2) INFORMATION FOR SEQ ID NO:52:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:52:
   AGGCAGACCC AAGGAAGCCA CACC 24
(2) INFORMATION FOR SEQ ID NO:53:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:53:
   AGGCTATCCC AGGAATTTGA CACC 24
(2) INFORMATION FOR SEQ ID NO:54:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:54:
   AGGAGAATCC TATGGGATAC ACC 23
(2) INFORMATION FOR SEQ ID NO:55:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:55:
   GGTGTTCATT GGGATAGCAT GCCT 24
(2) INFORMATION FOR SEQ ID NO:56:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:56:
   GGTGTTGGGA TTTCTGGATA GCCT 24
(2) INFORMATION FOR SEQ ID NO:57:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:57:
   AGGCGTTTGG GATGTATTTA CACC 24
(2) INFORMATION FOR SEQ ID NO:58:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:58:
   GGTGTGGGAT CGCCATATTC 20
(2) INFORMATION FOR SEQ ID NO:59:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:59:
   GGTGTGGGAT TGCTTTATTT 20
(2) INFORMATION FOR SEQ ID NO:60:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:60:
   GGTGTGGGAT TGGGACTAAA GCCTA 25
(2) INFORMATION FOR SEQ ID NO:61:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:61:
   GGTGTGGGAT TGGGACTAAA GCCT 24
(2) INFORMATION FOR SEQ ID NO:62:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:62:
   GGTGTAAGGA CAATGGGATA GCCT 24
(2) INFORMATION FOR SEQ ID NO:63:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:63:
   GGTGTCAGGA TTTGGGACAC GCCT 24
(2) INFORMATION FOR SEQ ID NO:64:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:64:
   GGTGTGGGAC TCAAAGAGGC 20
(2) INFORMATION FOR SEQ ID NO:65:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:65:
   GGTGTCCTCC AGCGGGATAA GCCT 24
(2) INFORMATION FOR SEQ ID NO:66:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:66:
   AGGCATCCGG GATAATAAAA CACC 24
(2) INFORMATION FOR SEQ ID NO:67:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:67:
   GGTGTTCTTC GGGATGGCTT GCCT 24
(2) INFORMATION FOR SEQ ID NO:68:
   (i) SEQUENCE CHARACTERISTICS:
      (A) LENGTH: 24 base pairs
      (B) TYPE: nucleic acid
      (C) STRANDEDMESS: single
      (D) TOPOLOGY: linear
   (ii) MOLECULE TYPE: cDNA
   (xi) SEQUENCE DESCRIPTION: SEQ ID NO:68:
   AGGCTTCACC GGGAGCACGA CACC 24
(2) INFORMATION FOR SEQ ID NO:69:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:69:
   GGTGTAGATC CCAGGGATTT GCCT 24
(2) INFORMATION FOR SEQ ID NO:70:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:70:
   GGTGTAGGTA GGGACATCCC GCCT 24
(2) INFORMATION FOR SEQ ID NO:71:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:71:
   GGTGTGAGAA ATAAGGGATA GCCT 24
(2) INFORMATION FOR SEQ ID NO:72:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:72:
   AGGCAAAAAA AAGGGGATAA CACC 24
(2) INFORMATION FOR SEQ ID NO:73:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:73:
   GGTGTGAAAT CTGAGGATCT GAAT 24
(2) INFORMATION FOR SEQ ID NO:74:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:74:
   NNTTGGGAWN NC 12
(2) INFORMATION FOR SEQ ID NO:75:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:75:
   AGGCTTTTGG GAATACCAGA CACC 24
(2) INFORMATION FOR SEQ ID NO:76:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:76:
   AGGCTTGGGA TTGGGAATAA CACC 24
(2) INFORMATION FOR SEQ ID NO:77:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:77:
   GGTGTTCCTG GGAATGTTCG GCCTA 25
(2) INFORMATION FOR SEQ ID NO:78:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:78:
   AGGCGTGGGA ATATCAGGAC ACC 23
(2) INFORMATION FOR SEQ ID NO:79:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:79:
   AGGCTGGGAA TGCTGGGAAA CACC 24
(2) INFORMATION FOR SEQ ID NO:80:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:80:
   GGTGTTGGGA ATGCTGGAAT GCCTA 25
(2) INFORMATION FOR SEQ ID NO:81:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:81:
   GGTGTAATTG GGAATTTTTA GCCTA 25
(2) INFORMATION FOR SEQ ID NO:82:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:82:
   GGTGTGGGAA AAGTGGGAAT GCCTA 25
(2) INFORMATION FOR SEQ ID NO:83:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:83:
   GGTGTTCCTG GGAATGCCAA GCCTA 25
(2) INFORMATION FOR SEQ ID NO:84:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:84:
   AGGCTACAGA ATACTGGGAA CACC 24
(2) INFORMATION FOR SEQ ID NO:85:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: CDNA
   (xi) SEQUENCE DESCRIPTION: SEQ ID NO:85:
   AGGCTAAAAA TTCCTGGGAA CACC 24
(2) INFORMATION FOR SEQ ID NO:86:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:86:
   AGGCATTCCC GTTTTGGGAA CACC 24
(2) INFORMATION FOR SEQ ID NO:87:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:87:
   AGGCATTCCC GTTTTGGGAA CACC 24
(2) INFORMATION FOR SEQ ID NO:88:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:88:
   GGTGTATCCC GGGAATACCG GCCTA 25
(2) INFORMATION FOR SEQ ID NO:89:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:89:
   AGGCTAAGGA ATACCGGGAA CACC 24
(2) INFORMATION FOR SEQ ID NO:90:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:90:
   AGGCTCTGGA ATATCGGGAA CACC 24
(2) INFORMATION FOR SEQ ID NO:91:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDKA
   (xi) SEQUENCE DESCRIPTION: SEQ ID NO:91:
   GGTGTAAATC GGGAATTCCG GCCTA 25
(2) INFORMATION FOR SEQ ID NO:92:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:92:
   AGGCCGGGAA TACCGGAAAA CACC 24
(2) INFORMATION FOR SEQ ID NO:93:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:93:
   AGGCAAAACA TTACAGGGAA CACC 24
(2) INFORMATION FOR SEQ ID NO:94:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:94:
   AGGCAGGGAA TATCGGGATA CACC 24
(2) INFORMATION FOR SEQ ID NO:95:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:95:
   GGTGTAGGAA TTCTAGGAAT GCCTA 25
(2) INFORMATION FOR SEQ ID NO:96:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:96:
   AGGCATTCCA AGGAATTTTA CACC 24
(2) INFORMATION FOR SEQ ID NO:97:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:97:
   GGTGTAAGGA ATACTGGAAT GCCTA 25
(2) INFORMATION FOR SEQ ID NO:98:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:98:
   GGCAGAATTC CAAGGAATAC ACC 23
(2) INFORMATION FOR SEQ ID NO:99:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:99:
   AGGCCAAGGA ATATCAGGAA CACC 24
(2) INFORMATION FOR SEQ ID NO:100:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDMA
   (xi) SEQUENCE DESCRIPTION: SEQ ID NO:100:
   TNHTGGGAAT DYY 13
(2) INFORMATION FOR SEQ ID NO:101:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:101:
   TTTTGGGAAT ACC 13
(2) INFORMATION FOR SEQ ID NO:102:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:102:
   TCAGCTTTTG GGAATCTCCT GTCA 24
(2) INFORMATION FOR SEQ ID NO:103:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:103:
   TGGAGGGAAG TGGGAAACTT TT 22
(2) INFORMATION FOR SEQ ID NO:104:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:104:
   TGGAAGTGGG AGGC 14
(2) INFORMATION FOR SEQ ID NO:105:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:105:
   GAGGAGAAAG GTCTCCTAC 19
(2) INFORMATION FOR SEQ ID NO:106:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:106:
   AACAGGGAAA CA 12
(2) INFORMATION FOR SEQ ID NO:107:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:107:
   GTCAGGGAAC AGG 13
(2) INFORMATION FOR SEQ ID NO:108:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:108:
   AAGGTGGGAA GTAA 14
(2) INFORMATION FOR SEQ ID NO:109:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:109:
   GGTAGGAATG G 11
(2) INFORMATION FOR SEQ ID NO:110:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:110:
   GGAGGGGGAA GAA 13
(2) INFORMATION FOR SEQ ID NO:111:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:111:
   AGTGGGGAAA TCT 13
(2) INFORMATION FOR SEQ ID NO:112:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:112:
   GGTCAGGGAA ACAA 14
(2) INFORMATION FOR SEQ ID NO:113:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:113:
   TGGGGGAAGG GGTGGAAG 18
(2) INFORMATION FOR SEQ ID NO:114:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:114:
   TTTTGGGAAC C 11
(2) INFORMATION FOR SEQ ID NO:115:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:115:
   AAAGGGGAAC CC 12
(2) INFORMATION FOR SEQ ID NO:116:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:116:
   TGGAGGGAG 9
(2) INFORMATION FOR SEQ ID NO:117:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:117:
   AGGGGAAA 8
(2) INFORMATION FOR SEQ ID NO:118:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:118:
   TTTGGGAATT 10
(2) INFORMATION FOR SEQ ID NO:119:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:119:
   TGAGAGGAAG AGGAGA 16
(2) INFORMATION FOR SEQ ID NO:120:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:120:
   CAGGAATT 8
(2) INFORMATION FOR SEQ ID NO:121:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:121:
   AAGGAAACCA AAACAGGGGA AG 22
(2) INFORMATION FOR SEQ ID NO:122:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:122:
   TTGGAAACCT 10
(2) INFORMATION FOR SEQ ID NO:123:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:123:
   GTTTCCATGA CATCATGAAT GGGAGT 26
(2) INFORMATION FOR SEQ ID NO:124:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:124:
   GTTTCCATGA TGTCATGAAT GGGGGT 26
(2) INFORMATION FOR SEQ ID NO:125:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:125:
   TTCTTGGGGA TTG 13
(2) INFORMATION FOR SEQ ID NO:126:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:126:
   GGAGGAACT 9
(2) INFORMATION FOR SEQ ID NO:127:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:127:
   TTTGGGATG 9
(2) INFORMATION FOR SEQ ID NO:128:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:128:
   TTCTAGGAAG TAAGGGAATT T 21
(2) INFORMATION FOR SEQ ID NO:129:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:129:
   GTGGGAAGA 9
(2) INFORMATION FOR SEQ ID NO:130:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:130:
   TAGGAATTCT 10
(2) INFORMATION FOR SEQ ID NO:131:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:131:
   TAAGGAAAGG 10
(2) INFORMATION FOR SEQ ID NO:132:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:132:
   TTTCCAAGTG GGAATC 16
(2) INFORMATION FOR SEQ ID NO:133:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:133:
   TGGGGAGTT 9
(2) INFORMATION FOR SEQ ID NO:134:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:134:
   TTGGGAAGGA T 11
(2) INFORMATION FOR SEQ ID NO:135:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:135:
   AAGGAACA 8
(2) INFORMATION FOR SEQ ID NO:136:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:136:
   CAGGGGAATC TCCCTCTCCA T 21
(2) INFORMATION FOR SEQ ID NO:137:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:137:
   AAGAGGAAAA 10
(2) INFORMATION FOR SEQ ID NO:138:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:138:
   GGGAAATTCC 10
(2) INFORMATION FOR SEQ ID NO:139:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:139:
   GGGGAATCCC 10
(2) INFORMATION FOR SEQ ID NO:140:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:140:
   TGGGAG 6
(2) INFORMATION FOR SEQ ID NO:141:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:141:
   CAGGGAAGTA 10
(2) INFORMATION FOR SEQ ID NO:142:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:142:
   CAAGGGACTT TCCGCTGGGG ACTTTCCAGG GAGGCG 36
(2) INFORMATION FOR SEQ ID NO:143:
   (i) SEQUENCE CHARACTERISTICS:
      (A) LENGTH: 103 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:143:
   TTTGGTTATA AATGTATTGA TTGCATCCCC ATTACCCAGA AGGCCAATAT TTAATTGGAG 60
   TCTTAACTCA ATTGTGTTTT CGTCAGTTGG TAAGCCTCAC AAA 103
(2) INFORMATION FOR SEQ ID NO:144:
   (i) SEQUENCE CHARACTERISTICS:
      (A) LENGTH: 116 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:144:
   ATGGGCCTTC CGGGCATGTA CCCAGGTAAG CACTGAGGCC CTGCTGAGCT GCACCCCTCC 60
   CCCTCCCAGC GCCTGGGCCA GGATGGGGCT CTGTGGCCTG TTTCAGCCAC AGGAGG 116
(2) INFORMATION FOR SEQ ID NO:145:
   (i) SEQUENCE CHARACTERISTICS:
      (A) LENGTH: 94 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:145:
   CCTTGTTGCT GCTGTGTTGC TATCTTGTGA CTTATTTTTG CAGTGACACT GAGTGGCCTC 60
   CTGTGTTGTC TCTTTCAGCC AGTAATGTTA AAGT 94
(2) INFORMATION FOR SEQ ID NO:146:
   (i) SEQUENCE CHARACTERISTICS:
      (A) LENGTH: 120 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:146:
   GAGCCCTGGC AGATGTGTCC TGTCTGCTGT GACACTAGAA CACCATTCAA CCCCTGGGTG 60
   TAGATTTCAC TTATGACCAT CTACTTCCCG CAGGAGACAA GTGCCTGTCA GACATGCCCT 120
(2) INFORMATION FOR SEQ ID NO:147:
   (i) SEQUENCE CHARACTERISTICS:
      (A) LENGTH: 120 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:147:
   ACATGTGTGG TTATCACAGC CAGGACAGGT ACGAGTTCTC ATCCCATATC ACGCGGGGGG 60
   AGCATCGTTA CCACCTGAGC TAAACCCAGC CAGGCCCCAC TGAAGCACAA AGATAGCTGG 120
(2) INFORMATION FOR SEQ ID NO:148:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:148:
   AGGAGGAAAA 10
(2) INFORMATION FOR SEQ ID NO:149:
   (i) SEQUENCE CHARACTERISTICS:
      (A) LENGTH: 7 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:149
   TGGGAAT 7
(2) INFORMATION FOR SEQ ID NO:150:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:150:
   TCAGCTTTTG GGAATCTCCT GTCA 24
(2) INFORMATION FOR SEQ ID NO:151:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:151:
   TCAGCTTTTG GGATTCCTCT CA 22
(2) INFORMATION FOR SEQ ID NO:152:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:152:
   TCAGCGGGGG GGAATACCCT GTCA 24

## Description

### Background of the Invention

The invention relates to the Ikaros gene and to the differentiation and generation of T cells.

The generation of the T cell repertoire from a progenitor stem cell proceeds through a differentiation pathway in which the later intrathymic steps are well documented while the early extrathymic events are only poorly characterized. One of the earliest definitive T cell differentiation markers is the CD3δ gene of the CD3/TCR complex.

Science, vol 258, 30 october 1992, pages 808-812 reports on Ikaros, an Early lymphoid-Specific Transcription Factor and a Portative Mediator for T Cell Commitment.

### Summary of the Invention

The Ikaros gene, a gene active in the early differentiation of T cells, has been discovered. The gene encodes a family of unique zinc finger proteins, the Ikaros proteins. The proteins of the Ikaros family are isoforms which arise from differential splicing of Ikaros gene transcripts. The isoforms of the Ikaros family generally include a common 3' exon (Ikaros exon E7, which includes amino acid residues 203-431 of mouse Ikaros) but differ in the 5' region. The Ikaros family includes all splicing variants which arise from transcription and processing of the Ikaros gene. Five isoforms are described herein. Ikaros proteins can bind and activate the enhancer of the CD3δ gene and are restricted primarily if not solely to T cells in the adult. The expression pattern of this transcription factor during embryonic development suggests that Ikaros proteins play a role as a genetic switch regulating entry into the T cell lineage. The Ikaros gene is also expressed in the proximal corpus striatum during early embryogenesis in mice.

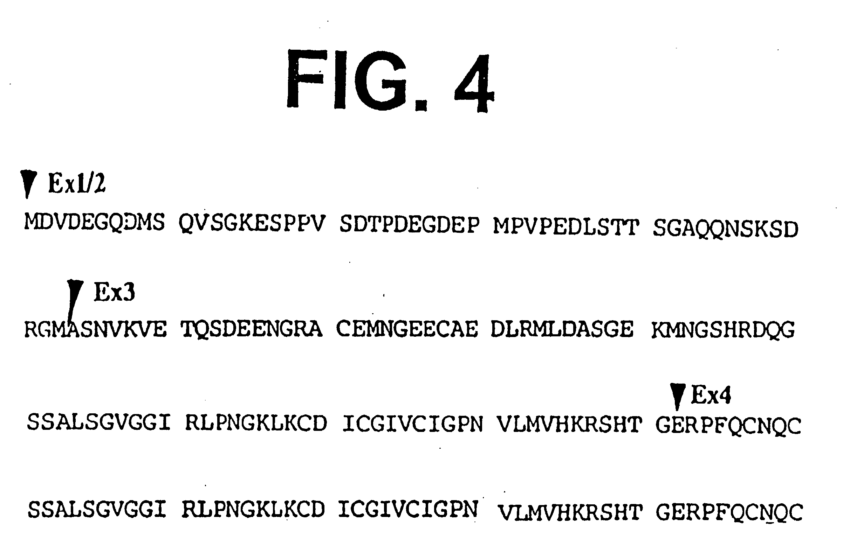

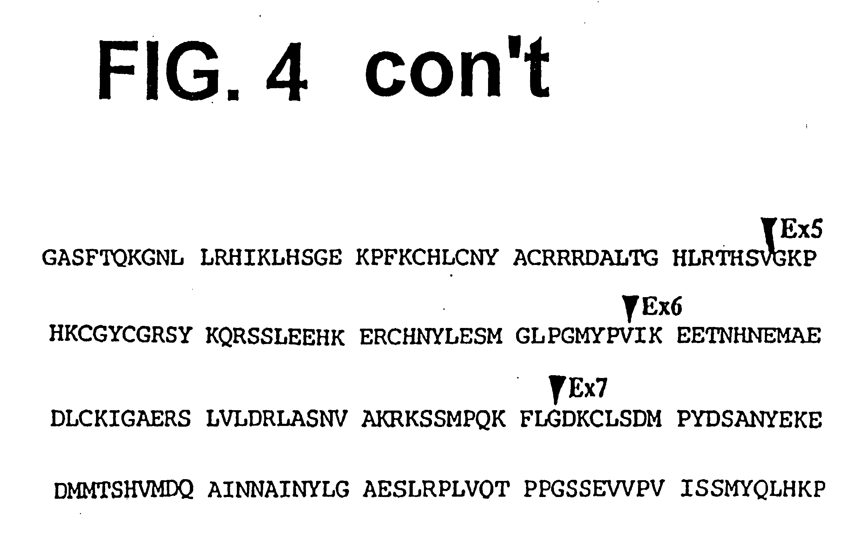

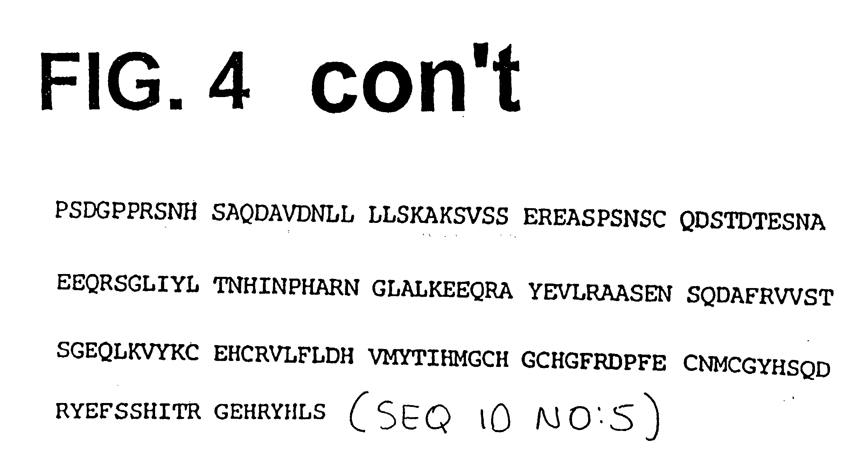

In general, the invention features, a DNA, preferably a purified DNA, including (or consisting of) a sequence which encodes a peptide including (or consisting of) one or more Ikaros exons of figure 4 (SEQ 10 NO:5) and which has an Ikaros activity. In preferred embodiments the Ikaros exon is any of E1/2, E3, E4, E5, E6, or E7; the purified DNA does not encode exon E7.

In other preferred embodiments: the encoded peptide further includes a second Ikaros exon; the second exon is any of E1/2, E3, E4, E5, E6, or E7; the first exon is E7 and the second exon is any of E1/2, E3, E4, E5, E6.

In other preferred embodiments: the encoded peptide further includes a third Ikaros exon; the third exon is any of E1/2, E3, E4, E5, E6, or E7; the first exon is E7, said second exon is E3, and the third exon is E1/2; the peptide is Ikaros isoform 5.

In other preferred embodiments: the encoded peptide further includes a fourth Ikaros exon; the fourth exon is any of E1/2, E3, E4, E5, E6, or E7; the first exon is E7, the second exon is E6, the third exon is E4, and the fourth exon is E1/2; the first exon is E7, the second exon is E4, the third exon is E3, and the fourth exon is E1/2; the peptide is Ikaros isoform 3 or 4.

In other preferred embodiments: the encoded peptide further includes a fifth Ikaros exon; the fifth exon is any of E1/2, E3, E4, E5, E6, or E7; the first exon is E7, the second exon is E6, the third exon is E5, the fourth exon is E4, and the fifth exon is E1/2; the peptide is Ikaros isoform 2.

In preferred embodiments: the encoded peptide further includes a sixth Ikaros exon; the sixth exon is any of E1/2, E3, E4, E5, E6, or E7; the first exon is E7, the second exon is E6, the third exon is E5, the fourth exon is E4, the fifth exon is E3, and the sixth exon is E1/2; the peptide is Ikaros isoform 1.

In preferred embodiments: the sequence of the encoded Ikaros exon is the same as that of a naturally occurring Ikaros exon, or a fragment thereof having Ikaros activity; the DNA sequence which encodes the Ikaros exon is at least 85%, more preferably at least 90%, yet more preferably at least 95%, and most preferably at least 98 or 99% homologous with DNA encoding a naturally occurring Ikaros exon, or a fragment thereof having Ikaros activity, e.g., Ikaros exon encoding DNA from SEQ ID NO:2 or SEQ ID NO:3; the sequence which encodes an Ikaros exon hybridizes under high or low stringency to a nucleic acid which encodes a naturally occurring Ikaros exon, or a fragment thereof having Ikaros activity, e.g., an Ikaros exon with the same amino acid sequence as an Ikaros exon of SEQ ID NO:2, SEQ ID NO:3, or SEQ ID NO:5; the amino acid sequence of the encoded Ikaros exon is at least 30, more preferably at least 40, more preferably at least 50, and most preferably at least 60, 80, 100, or 200 amino acid residues in length; the encoded Ikaros amino acid sequence is at least 50% more preferably 60%, more preferably 70%, more preferably 80%, more preferably 90%, and most preferably 95% as long as a naturally occurring Ikaros exon, or a fragment thereof having Ikaros activity; the encoded Ikaros exon is equal in length to a naturally occurring Ikaros exon, or a fragment thereof having Ikaros activity; the amino acid sequence of the encoded Ikaros exon is at least 80%, more preferably at least 85%, yet more preferably at least 90%, yet more preferably at least 95%, and a most preferably at least 98 or 99% homologous with a naturally occurring Ikaros exon sequence, or a fragment thereof having Ikaros activity, e.g., an Ikaros exon sequence of SEQ ID NO:2, SEQ ID NO:3, or SEQ ID NO:5; the encoded Ikaros exon amino acid sequence is the same as that of a naturally occurring Ikaros exon, or a fragment of the sequence thereof, e.g., an Ikaros exon described in SEQ ID NO:2, SEQ ID NO:3, or SEQ ID NO:5; and the peptide has Ikaros peptide activity.

In preferred embodiments: the exons in the encoded peptide are arranged in the same relative linear order as found in a naturally occurring isoform, e.g., Ikaros isoform 1, e.g., in a peptide having the exons E3 and E7, E 3 is located N-terminal to E7; the linear order of the encoded exons is different from that found in a naturally occurring isoform, e.g., in Ikaros isoform 1, e.g., in a peptide having exons E3, E5, and E7, the direction N-terminal to C-terminal end, is E5, E3, E7; the exons in the encoded peptide differ in one or more of composition (i.e., which exons are present), linear order, or number (i.e., how many exons are present or how many times a given exon is present) from a naturally occurring Ikaros isoform, e.g., from Ikaros isoform 1, 2, 3, 4, or 5.

In another aspect, the invention features, a peptide, preferably a pure peptide, including (or consisting of) one or more Ikaros exons. In preferred embodiments the Ikaros exon is E1/2, E3, E4, E5, E6, or E7; the peptide does not include exon E7.

In other preferred embodiments: the peptide further includes a second Ikaros exon; the second exon is any of E1/2, E3, E4, E5, E6, or E7; the first exon is E7 and the second exon is any of E1/2, E3, E4, E5, E6.

In other preferred embodiments: the peptide further includes a third Ikaros exon; the third exon is any of E1/2, E3, E4, E5, E6, or E7; the first exon is E7, the second exon is E3, and the third exon is E1/2; the peptide is Ikaros isoform 5.

In other preferred embodiments: the peptide further includes a fourth Ikaros exon; the fourth exon is any of E1/2, E3, E4, E5, E6, or E7; the first exon is E7, the second exon is E4, the third exon is E3, and the fourth exon is E1/2; the first exon is E7, the second exon is E4, the third exon is E3, and the fourth exon is E1/2; the peptide is Ikaros isoform 3 or 4..

In other preferred embodiments the peptide further includes a fifth Ikaros exon; the fifth exon is any of E1/2, E3, E4, E5, E6, or E7; the first exon is E7, the second exon is E6, the third exon is E5, the fourth exon is E4, and the fifth exon is E1/2; the peptide is Ikaros Isoform 2.

In other preferred embodiments the peptide further includes a sixth Ikaros exon; the sixth exon is any of E1/2, E3, E4, E5, E6, or E7; the first exon is E7, the second exon is E6, the third exon is E5, the fourth exon is E4, the fifth exon is E3, and the sixth exon is E1/2; the peptide is Ikaros isoform 1.

In preferred embodiments: the sequence of the Ikaros exon is the same as that of a naturally occurring Ikaros exon, or a fragment thereof having Ikaros activity; the amino acid sequence of the Ikaros exon is such that a nucleic acid sequence which encodes it is at least 85%, more preferably at least 90%, yet more preferably at least 95%, and most preferably at least 98 or 99% homologous with DNA encoding a naturally occurring Ikaros exon, or a fragment thereof having Ikaros activity, e.g., Ikaros exon encoding DNA from SEQ ID NO:2 or SEQ ID NO:3; the amino acid sequence of the Ikaros exon is such that a nucleic acid sequence which encodes it hybridizes under high or low stringency to a nucleic acid which encodes a naturally occurring Ikaros exon, or a fragment thereof having Ikaros activity, e.g., an Ikaros exon with the same amino acid sequence as an Ikaros exon of SEQ ID NO:2, SEQ ID NO:3, or SEQ ID NO:5; the amino acid sequence of the Ikaros exon is at least 30, more preferably at least 40, more preferably at least 50, and most preferably at least 60, 80, 100, or 200 amino acid residues in length; the encoded Ikaros amino acid sequence is at least 50% more preferably 60%, more preferably 70%, more preferably 80%, more preferably 90%, and most preferably 95% as long as a naturally occurring Ikaros exon, or a fragment thereof having Ikaros activity; the Ikaros exon is equal in length to a naturally occurring Ikaros exon; the amino acid sequence of the Ikaros exon is at least 80%, more preferably at least 85%, yet more preferably at least 90%, yet more preferably at least 95%, and a most preferably at least 98 or 99% homologous with a naturally occurring Ikaros exon sequence, or a fragment thereof having Ikaros activity, e.g., an Ikaros exon sequence of SEQ ID NO:2, SEQ ID NO:3, or SEQ ID NO:5; the Ikaros exon amino acid sequence is the same as that of a naturally occurring Ikaros exon, or a fragment of the sequence thereof, e.g., an Ikaros exon described in SEQ ID NO:2, SEQ ID NO:3, or SEQ ID NO:5; and the peptide has Ikaros peptide activity.

In preferred embodiments: the exons in the peptide are arranged in the same relative linear order as found in a naturally occurring isoform, e.g., in Ikaros isoform 1, e.g., in a peptide having the exons E3 and E7, E 3 is located N-terminal to E7; the linear order of the exons is different from that found in a naturally occurring isoform, e.g., in Ikaros isoform 1, e.g., in a peptide having exons E3, E5, and E7, the direction N-terminal to C-terminal end, is E5, E3, E7; the exons in the peptide differ in one or more of composition (i.e., which exons are present), linear order, or number (i.e., how many exons are present or how many times a given exon is present) from a naturally occurring Ikaros isoform, e.g., from Ikaros isoform 1, 2, 3, 4, or 5.

In another aspect, the invention features, a DNA, preferably a purified DNA, which includes (or consists of) a DNA sequence encoding an Ikaros peptide, e.g., an Ikaros peptide having Ikaros activity, e.g., Ikaros isoform 1, 2, 3, 4, or 5. In preferred embodiments: the sequence of the encoded Ikaros peptide is the same as the sequence of a naturally occurring Ikaros peptide, or a fragment thereof having Ikaros activity; the DNA sequence is at least 85%, more preferably at least 90%, yet more preferably at least 95%, and most preferably at least 98 or 99% homologous with DNA encoding a naturally occurring Ikaros peptide, or a fragment thereof having Ikaros activity; e.g., with DNA from SEQ ID NO:2 or SEQ ID NO:3; the amino acid sequence of the encoded peptide is such that it can be encoded by a nucleic acid which hybridizes under high or low stringency conditions to a nucleic acid which encodes a peptide with the same, amino acid sequence as the peptide of SEQ ID NO:2, SEQ ID NO:3, or SEQ ID NO:5; the encoded peptide is at least 30, more preferably at least 40, more preferably at least 50, and most preferably at least 60, 80, 100, or 200 amino acid residues in length; the encoded peptide is at least 50% more preferably at least 60%, more preferably 70%, more preferably 80%, more preferably 90%, and most preferably 95% as long as a naturally occurring Ikaros peptide, or a fragment thereof having Ikaros activity; the encoded peptide is the same length as a naturally occurring Ikaros peptide, or a fragment thereof having Ikaros activity; the encoded peptide is at least 80%, more preferably at least 85%, yet more preferably at least 90%, yet more preferably at least 95%, and a most preferably at least 98 or 99% homologous with an amino acid sequence which is the same as a naturally occurring Ikaros peptide, or a fragment thereof having Ikaros activity, e.g., the peptide sequence of SEQ ID NO:2, SEQ ID NO:3, or SEQ ID NO:5; and, the amino acid sequence of the peptide is the same as the sequence of a naturally occurring Ikaros peptide, or a fragment thereof having Ikaros activity, e.g., the sequence, described in SEQ ID NO:2, SEQ ID NO:3, or SEQ ID NO:5.

In another aspect, the invention features, a DNA, preferably a purified DNA, which includes (or consists of) a sequence encoding a peptide of 20 or more amino acids in length, the peptide having at least 90% homology with an amino acid sequence which is the same as a naturally occurring Ikaros peptide, e.g., the amino acid sequence of SEQ ID NO:2, SEQ ID NO:3, or SEQ ID NO:5. In preferred embodiments the purified DNA encodes: a peptide which is at least 30, more preferably at least 40, more preferably at least 50, and most preferably at least 60, 80, 100, or 200, amino acid residues in length; the encoded peptide is at least 50% more preferably at least 60%, more preferably 70%, more preferably 80%, more preferably 90%, and most preferably 95% as long as a naturally occurring Ikaros peptide, or fragment thereof having Ikaros activity; the encoded peptide is the same length as a naturally occurring Ikaros peptide; a peptide which is at least 80, more preferably at least 85, yet more preferably at least 90, yet more preferably at least 95, and most preferably at least 98 or 99% homologous with an amino acid sequence which is the same as a naturally occurring Ikaros peptide, or a fragment thereof having Ikaros activity, e.g., as the amino acid sequence of SEQ ID NO:2, SEQ ID NO:3, or SEQ ID NO:5; and, a peptide with Ikaros activity.

In another aspect, the invention features, a DNA, preferably a purified DNA, which includes (or consists of) a DNA sequence which hybridizes under high or low stringency to a nucleic acid which encodes a peptide with the same amino acid sequence as a naturally occurring Ikaros peptide, e.g., the peptide of SEQ ID NO:2, SEQ ID NO:3, or SEQ ID NO:5. In preferred embodiments: the DNA sequence is at least 85%, more preferably at least 90%, yet more preferably at least 95%, and most preferably at least 98 or 99% homologous with DNA encoding a naturally occurring Ikaros peptide, or a fragment thereof having Ikaros activity, e.g., with DNA from of SEQ ID NO:2 or SEQ ID NO:3; the purified DNA encodes a peptide at least 30, more preferably at least 40, more preferably at least 50, and most preferably at least 60, 80, 100, or 200 amino acid residues in length; the encoded peptide is at least 50% more preferably at least 60%, more preferably 70%, more preferably 80%, more preferably 90%, and most preferably 95% as long a naturally occurring Ikaros peptide, or fragment thereof having Ikaros activity; the encoded peptide is the same length as a naturally occurring Ikaros peptide; the purified DNA encodes a peptide at least 80, more preferably at least 85, yet more preferably at least 90, yet more preferably at least 95, and most preferably at least 98 or 99% homologous with an amino acid sequence which is the same as a naturally occurring Ikaros peptide, e.g., the amino acid sequence of SEQ ID NO:2, SEQ ID NO:3, or SEQ ID NO:5; and, the purified DNA encodes a peptide having the same amino acid sequence, or a fragment of the amino acid sequence, described in SEQ ID NO:2, SEQ ID NO:3, or SEQ ID NO:5.

In another aspect, the invention includes a vector which includes DNA of the invention, preferably a purified DNA of the invention, which encodes a peptide of the invention.

The invention also includes: a cultured cell or an hematopoietic stem cell, containing purified Ikaros-protein-encoding-DNA; a cell capable of expressing an Ikaros protein; a cell capable of giving rise to a transgenic animal or to a homogeneous population of hemopoietic cells, e.g., lymphoid cells, e.g., T cells; a homogeneous population of cells, each of which includes purified Ikaros-protein-encoding-DNA; and a method for manufacture of a peptide of the invention including culturing a cell which includes a DNA, preferably a purified DNA, of the invention in a medium to express the peptide.

In another aspect, the invention features a peptide of the invention, preferably a pure peptide of the invention, e.g.: a peptide having Ikaros activity, e.g., Ikaros isoform 1, 2, 3, 4, or 5. In preferred embodiments: the sequence of the encoded Ikaros peptide is the same as the sequence of a naturally occurring Ikaros peptide, or a fragment thereof having Ikaros activity; the sequence of the peptide is such that it is encoded by a DNA sequence at least 85%, more preferably at least 90%, yet more preferably at least 95%, and most preferably at least 98 or 99% homologous with DNA encoding a naturally occurring Ikaros peptide, or a fragment thereof having Ikaros activity; e.g., with DNA from SEQ ID NO:2 or SEQ ID NO:3; the amino acid sequence of the peptide having Ikaros activity is such that it can be encoded by a nucleic acid which hybridizes under high or low stringency conditions to a nucleic acid which encodes a peptide with the same amino acid sequence as the peptide of SEQ ID NO:2, SEQ ID NO:3, or SEQ ID NO:5; the peptide is at least 30, more preferably at least 40, more preferably at least 50, and most preferably at least 60, 80, 100, or 200 amino acid residues in length; the peptide is at least 50% more preferably at least 60%, more preferably 70%, more preferably 80%, more preferably 90%, and most preferably 95% as long as a naturally occurring Ikaros peptide, or fragment thereof having Ikaros activity; the peptide is the same length as a naturally occurring Ikaros peptide, or a fragment thereof having Ikaros activity; the peptide is at least 80%, more preferably at least 85%, yet more preferably at least 90%, yet more preferably at least 95%, and a most preferably at least 98 or 99% homologous with an amino acid sequence which is the same as a naturally occurring Ikaros peptide, or a fragment thereof having Ikaros activity, e.g., the peptide sequence of SEQ ID NO:2, SEQ ID NO:3, or SEQ ID NO:5; and, the amino acid sequence of the peptide is the same as the sequence of a naturally occurring Ikaros peptide, or a fragment thereof having Ikaros activity, e.g., the sequence, described in SEQ ID NO:2, SEQ ID NO:3, or SEQ ID NO:5.

In preferred embodiments a peptide of the invention, preferably a purified peptide of the invention, is produced by expression of a DNA of the invention, preferably a purified DNA of the invention.

The invention also includes pure preparation of an antibody, preferably a monoclonal antibody directed against an Ikaros protein; a therapeutic composition including an Ikaros protein and a pharmaceutically acceptable carrier; a therapeutic composition which includes a purified DNA of the invention and a pharmaceutically acceptable carrier.

In another aspect, the invention permits a method for treating an animal, e.g., a human, a mouse, a transgenic animal, or an animal model for an immune system disorder, e.g., a T or B cell related disorder, e.g., a nude mouse or a SCID mouse, including administering a therapeutically-effective amount of an Ikaros peptide to the animal.

In another aspect, the invention permits a method for treating an animal, e.g., a human, a mouse, a transgenic animal, or an animal model for an immune system disorder, e.g., a T or B cell related disorder, e.g., a nude mouse or a SCID mouse including administering to the animal cells selected, e.g., selected in vitro, for the expression of a product of the Ikaros gene, e.g., hematopoietic stem cells, e.g., cells transformed with Ikaros-peptide-encoding DNA, e.g., hematopoietic stem cells transformed. with Ikaros-peptide-encoding DNA.

In preferred embodiments: the cells are taken from the animal to which they are administered; the cells are taken from an animal which is MHC matched with the animal to which they are administered; the cells are taken from an animal which is syngeneic with the animal to which they are administered; the cells are taken from an animal which is of the same species as is the animal to which they are administered.

In another aspect, the invention permits a method for treating an animal, e.g., a human, a mouse, a transgenic animal, or an animal model for an immune system disorder, e.g., a T or B cell related disorder, e.g., a nude mouse or a SCID mouse, including administering to the animal a nucleic acid encoding an Ikaros peptide and expressing the nucleic acid.

In another aspect, the invention permits a method of evaluating the effect of a treatment, e.g., a treatment designed to promote or inhibit hematopoiesis, including carrying out the treatment and evaluating the effect of the treatment on the expression of the Ikaros gene.

In preferred embodiments the treatment is administered: to an animal, e.g., a human, a mouse, a transgenic animal, or an animal model for an immune system disorder, e.g., a T or B cell related disorder, e.g., a nude mouse or a SCID mouse, or a cell, e.g., a cultured hematoporetic stem cell.

In another aspect, the invention permits a method for determining if a subject, e.g., a human, is at risk for a disorder related to mis-expression of the Ikaros gene, e.g., a leukemic disorder or other disorder of the immune system, e.g., an immunodeficiency, or a T or B cell related disorder, e.g., a disorder characterized by a shortage of T or B cells, including examining the subject for the expression of the Ikaros gene, non-wild type expression or mis-expression being indicative of risk.

In another aspect, the invention features a method for determining if a subject, e.g., a human, is at risk for a disorder related to mis-expression of the Ikaros gene, e.g., a leukemic disorder or other disorder of the immune system, e.g., an immunodeficiency, or a T or B cell related disorder, e.g., a disorder characterized by a shortage of T or B cells, including providing a nucleic acid sample from the subject and determining if the structure of an Ikaros gene allele of the subject differs from wild type.

In preferred embodiments: the determination includes determining if an Ikaros gene allele of the subject has a gross chromosomal rearrangement; the determination includes sequencing the subject's Ikaros gene.

In another aspect, the invention permits, a method of evaluating an animal or cell model for an immune disorder, e.g., a T cell related disorder, e.g., a disorder characterized by a shortage of T or B cells, including determining if the Ikaros gene in the animal or cell model is expressed at a predetermined level or if the Ikaros gene is mis-expressed. In preferred embodiments: the predetermined level is lower than the level in a wild type or normal animal; the predetermined level is higher than the level in a wild type or normal animal; or the pattern of isoform expression is altered from wildtype.

In another aspect, the invention features a transgenic rodent, e.g., a mouse, having a transgene which includes an Ikaros gene or Ikaros protein encoding DNA. In preferred embodiments: the Ikaros gene or DNA includes a deletion, e.g. a deletion of all or part of one or more Ikaros exons, e.g., a deletion of all or part of exon E7 or a deletion of all or part of exons E3 or E4, or is otherwise mis-expressed.

In another aspect the invention features a method of expressing a heterologous gene, e.g., in a culthred cell e.g., a hematopoietic stem cell, including placing the gene under the control of an Ikaros-responsive control element, and contacting the Ikaros-responsive control element with an Ikaros protein.

In preferred embodiments: the Ikaros-responsive control element includes an enhancer, e.g., an δA element, an NFKB element, or one of the Ikaros binding sequences, e.g., one of the consensus sequences, disclosed herein; the Ikaros-responsive control element includes the regulatory region of the CD3δ gene; the heterologous gene and the Ikaros-responsive control element are carried on a vector; the method further includes the step of transforming a cell with a vector which includes a heterologous gene under the control of an Ikaros-responsive control agent; the heterologous gene is expressed in a cell which normally includes or expresses an Ikaros protein.

In another aspect, the invention features a method of expressing a gene under the control of an Ikaros-responsive control element in a cell including administering an Ikaros protein to the cell.

In preferred embodiments: the method further includes transforming the cell with DNA which encodes an Ikaros protein to supply an Ikaros protein; the gene is a heterologous gene.

In another aspect, the invention permits a method for treating an animal, e.g., a human, a mouse, a transgenic animal, or an animal model for a disorder of the nervous system, e.g., a disorder of the corpus striatum, e.g., Alzheimer's disease, immune system disorder, including administering a therapeutically effective amount of an Ikaros protein to the animal.

In another aspect, the invention permits a method for treating an animal, e.g., a human, a mouse, a transgenic animal, or an animal model for a disorder of the nervous system, e.g., a disorder of the corpus striatum, e.g., Alzheimer's disease, including administering to the animal cells selected, e.g., selected in vitro, for the expression of a product of the Ikaros gene, e.g., hematopoietic stem cells, e.g., cells transformed with Ikaros-protein-encoding DNA, e.g., hematopoietic stem cells transformed with Ikaros-protein-encoding DNA.

In preferred embodiments: the cells are taken from the animal to which they are administered; the cells are taken from an animal which is MHC matched with the animal to which they are administered; the cells are taken from an animal which is syngeneic with the animal to which they are administered: the cells are taken from an animal which is of the same species as is the animal to which they are administered.

In another aspect, the invention permits a method for treating an animal, e.g., a human, a mouse, a transgenic animal, or an animal model for a disorder of the nervous system, e.g., a disorder of the corpus striatum, e.g., Alzheimer's disease, including administering to the animal a nucleic acid encoding an Ikaros peptide and expressing the nucleic acid.

In another aspect, the invention permits a method of evaluating the effect of a treatment for a disorder of the nervous system, e.g., a disorder of the corpus striatum, e.g., Alzheimer's disease, including administering the treatment and evaluating the effect of the treatment on the expression of the Ikaros gene.

In preferred embodiments the treatment is administered: to an animal, e.g., a human, a mouse, a transgenic animal, or an animal model for a disorder of the nervous system, e.g., a disorder of the corpus striatum, e.g., Alzheimer's disease, or a cell, e.g., a cultured hematopoietic stem cell.

In another aspect, the invention permits a method for determining if a subject, e.g., a human, is at risk for a disorder related to mis-expression of the Ikaros gene, e.g., a disorder of the nervous system, e.g., a disorder of the corpus striatum, e.g., Alzheimer's disease, including examining the subject for the expression of the Ikaros gene, non-wild type expression or mis-expression being indicative of risk.

In another aspect, the invention features a method for determining if a subject, e.g., a human, is at risk for a disorder related to mis-expression of the Ikaros gene, e.g., a disorder of the nervous system, e.g., a disorder of the corpus striatum, e.g., Alzheimer's disease, including providing a nucleic acid sample from the subject and determining if the structure of an Ikaros gene allele of the subject differs from wild type.

In preferred embodiments: the determination includes determining if an Ikaros gene allele of the subject has a gross chromosomal rearrangement; the determination includes sequencing the subject's Ikaros gene.

In another aspect, the invention permits a method of evaluating an animal or cell model for a disorder of the nervous system, e.g., a disorder of the corpus striatum, e.g., Alzheimer's disease, including determining if the Ikaros gene in the animal or cell model is expressed at a predetermined level or if the Ikaros gene is mis-expressed.

In preferred embodiments: the predetermined level is lower than the level in a wild type or normal animal; the predetermined level is higher than the level in a wild type or normal animal.

In another aspect, the invention features, a method of inhibiting an interaction, e.g., binding, between a protein, e.g., a first Ikaros isoform, and a DNA sequence, e.g. a DNA sequence under the control of a δA sequence, an NKFB sequence, a sequence which corresponds to an Ikaros binding oligonucleotide described herein, or a site present in the control region of a lymphocyte restricted gene, e.g., TCR-α, -β, or -δ, CD3 -δ, -ε, -γ genes, the SL3 gene, or the HIV LTR gene. The methods includes contacting the DNA sequence with an effective amount of a second Ikaros isoform, or with a DNA binding fragment of an Ikaros isoform, e.g., of the second Ikaros isoform.

In preferred embodiments the fragment is deleted for all or part of an Ikaros exon, e.g., for all or part of E1/2, E3, E4, E5, E6, or E7.

In another aspect, the invention features, a method of inhibiting an interaction, e.g., binding, between a protein, e.g., a first Ikaros isoform, and a DNA sequence, e.g., a δA sequence, an NKFB sequence, a sequence which corresponds to an Ikaros binding oligonucleotide described herein, or a site present in the control region of a lymphocyte restricted gene, e.g., TCR-α, -β, or -δ, CD3 -δ, -ε, -γ genes, the SL3 gene, or the HIV LTR gene. The methods includes contacting the protein with an effective amount of an Ikaros binding oligonucleotide. In preferred embodiments the oligonucleotide includes a sequence chosen from, IK-BS1, IK-BS2, IK-BS3, IK-BS4, or IK-BS5.

In preferred embodiments: the oligonucleotide preferentially binds to a first Ikaros isoform; the oligonucleotide preferentially binds to a second Ikaros isoform.

In another aspect the invention includes an Ikaros binding oligonucleotide, e.g., IK-BS1, IK-BS2, IK-BS3, IK-BS4, or IK-BS5. In preferred embodiments the oligonucleotide contains at least two, three, four, or five copies of one of the Ikaros binding oligonucleotide sequences disclosed herein.

In another aspect, the invention features a method of attenuating the binding of a first Ikaros isoform to target DNA. The method includes contacting the target DNA with an effective amount of a second Ikaros isoform, or with a DNA binding fragment of said second isoform.

Heterologous gene, as used herein, is a gene which is not normally under the control of an Ikaros responsive control element.

An Ikaros-responsive control element, as used herein is a region of DNA which, when present upstream or downstream from a gene, results in regulation, e.g., increased transcription of the gene in the presence of an Ikaros protein.

Purified DNA is DNA that is not immediately contiguous with both of the coding sequences with which it is immediately contiguous (i.e., one at the 5' end and one at the 3' end) in the naturally occurring genome of the organism from which the DNA of the invention is derived. The term therefore includes, for example, a recombinant DNA which is incorporated into a vector; into an autonomously replicating plasmid or virus; or into the genomic DNA of a prokaryote or eukaryote, or which exists as a separate molecule (e.g., a cDNA or a genomic DNA fragment produced by PCR or restriction endonuclease treatment) independent of other DNA sequences. It also includes a recombinant DNA which is part of a hybrid gene encoding additional polypeptide sequence.

Homologous refers to the sequence similarity between two polypeptide molecules or between two nucleic acid molecules. When a position in both of the two compared sequences is occupied by the same base or amino acid monomeric subunit, e.g., if a position in each of two DNA molecules is occupied by adenine, then the molecules are homologous at that position. The homology between two sequences is function of the number of matching or homologous positions shared by the two sequences. For example, 6 of 10, of the positions in two sequences are matched or homologous then the two sequences are 60% homologous. By way of example, the DNA sequences ATTGCC and TATGGC share 50% homology.

A transgene is defined as a piece of DNA which is inserted by artifice into a cell and becomes a part of the genome of the animal which develops in whole or part from that cell. Such a transgene may be partly or entirely heterologous to the transgenic animal.

A transgenic animal, e.g., a transgenic mouse, is an animal having cells that contain a transgene, which transgene was introduced into the animal, or an ancestor of the animal, at a prenatal, e.g., an embryonic stage.

An enhancer region is defined as a cis-acting DNA sequence capable of increasing transcription from a promoter that is located either upstream or downstream of the enhancer region. Such DNA sequences are well known to those skilled in the art of eukaryotic gene expression.

A pure preparation of a peptide is a preparation which is free of the peptides with which it naturally occurs in a cell. A pure preparation of a non-naturally occuring peptide is one wich is at least 10% by weight of the peptide of interest.

Mis-expression, as used herein, refers to a non-wild type pattern of gene expression. It includes: expression at non-wild type levels, i.e., over or under expression; a pattern of expression that differs from wild type in terms of the time or stage at which the gene is expressed, e.g., increased or decreased expression (as compared with wild type) at a predetermined developmental period or stage; a pattern of expression that differs from wild type in terms of the tissue specificity of expression, e.g., increased or decreased expression (as compared with wild type) in a predetermined cell type or tissue type; a pattern of expression that differs from wild type in terms of the size, amino acid sequence, post-translational modification, or a biological activity of an Ikaros gene product; a pattern of expression that differs from wild type in terms of the effect of an environmental stimulus or extracellullar stimulus on expression of the gene, e.g., a pattern of increased or decreased expression (as compared with wild type) in the presence of an increase or decrease in the strength of the stimulus; or a pattern of isoform expression which differs from wildtype.

The terms peptide, protein, and polypeptide are used interchangeably herein.

A peptide has Ikaros activity if it has one or more of the following properties: the ability to stimulate transcription of a DNA sequence under the control any of a δA element, an NFKB element, or one of the Ikaros binding oligonucleotide consensus sequences disclosed herein; the ability to bind to any of a δA element, an NFKB element, or one of the Ikaros binding oligonucleotide consensus sequences disclosed herein; or the ability to competitively inhibit the binding of a naturally occurring Ikaros isoform to any of a δA element, an NFKB element, or one of the Ikaros binding oligonucleotide consensus sequences disclosed herein. An Ikaros peptide is a peptide with Ikaros activity.

The invention is useful for identifying T cells; identifying cells which can develop into T cells; and generally, in the investigation of hemopoiesis, e.g., in the differentiation of progenitor hematopoietic stem cells into T cells. The role of the Ikaros gene and its products can be studied, e.g., in cells, e.g., cultured cells, transformed with the Ikaros gene or fragments thereof, or in transgenic animals. The invention is also useful for: promoting the expression of markers of cell lineage, e.g., CD3δ genes; enhancing the ability of a cell, e.g., a hematopoietic stem cell, to develop into a T cell; screening individuals at risk for genetic T cell disorders, e.g., leukemia; and treating immune disorders (e.g., immunodeficiencies, e.g., AIDS, or chemical, drug, or radiation induced immunodeficiencies, or cancers, e.g., leukemia) characterized by a shortage of T cells; for investigating the structure and expression of the Ikaros gene or iso forms of the gene product; for investigating species or tissue differences in the expression of the Ikaros gene or its isoforms; for investigating the structure and function of DNA binding proteins; for studying the structure and function of zinc finger containing proteins; for the construction of transgenic animals; for inhibiting the binding of Ikaros to a target molecule; for studying the relative affinities of Ikaros isoforms for target DNA; and for searching for or manipulating the expression of genes under the control of Ikaros isoforms.

Other features and advantages of the invention will be apparent from the following description and from the claims.

### Detailed Description

The drawings are first briefly described.

### Drawings

Fig. 1A is a map of the δA element of the CD3 enhancer (SEQ ID NO:1).

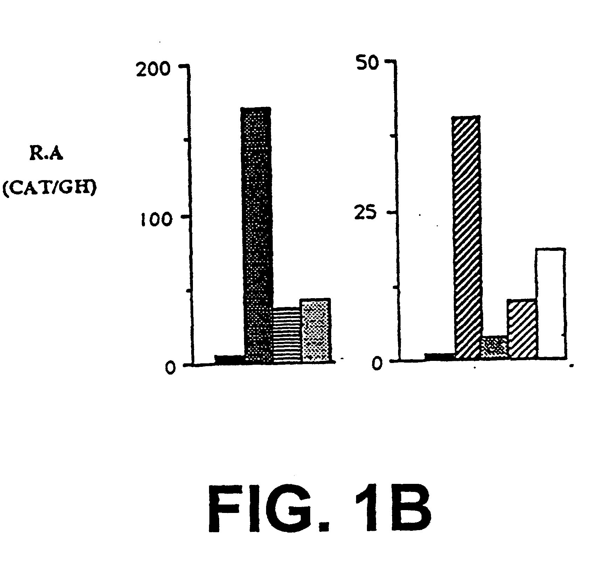

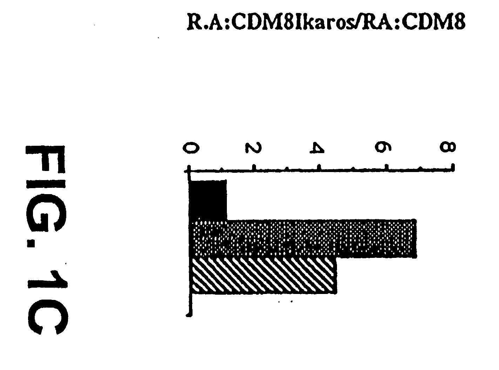

Fig. 1B is a graph of the contribution of the CRE and the G box to the activity of the element as analyzed by expression of tkCAT reporter gene under the control of various element sequences. In the bar graph on the left, the solid bar represents the tkcat reporter gene, the shaded bar represents tkcat3δA, the horizontal line bar represents tkcat3δAmu1(-CRE), and the stippled bar represents tkcat3δAmu2(-Gbox). In the bar graph on the right, the solid bar represents tkcat, the diagonal bar on the left of the graph is tkcatδehn, the stippled bar is tkcatδenh(-CRE), the diagonal bar on the right is tkcatδenh(-δA), and the clear box represents tkcatδenh(-Gbox).

Fig. 1C is a graph of the effect of Ikaros expression on the activity of the δ element in non-T cells. In the bar graph, the solid bar represents tkcat, the stippled bar represents tkcat3δA, and the cross-hatched bar represents tkcatδenh.

Fig. 2 is a map of the DNA sequence of a murine Ikaros cDNA and the desired amino acid sequence encoded thereby (SEQ ID NO:2).

Fig. 3 is a partial sequence of a human Ikaros cDNA (SEQ ID NO:3).

Fig. 4 is a depiction of the amino acid composition of the IK- 1 cDNA( SEQ ID NO:5). (IK-1 is comprised of all six coding exons (Ex1/2; Ex3; Ex4; Ex5; Ex6; and Ex7.) An arrow indicates the first amino acid in every exon.

Fig. 5 is a diagram of exon usage in the Ikaros 1-5 cDNAs. Exon numbers are indicated at the bottom left hand comer of each box (Ex). Zinc finger modules are shown on top of the encoding exons (Fx). The first row represents the Ikaros 1, the second row the Ikaros 2, the third row the Ikaros 3, the fourth row the Ikaros 4, and the fifth row the Ikaros 5.

Fig. 6 is a depiction of the exon organization at the Ikaros locus indicating primer sets 1/2 and 3/4 used for amplification of the respective isoforms. Specifically, Figure 6 represents Oligo1/2 IK-1/IK-2/IK-4 and Oligo3/4 IK-1/IK-3/IK-5.

Fig. 7 is a map of the genomic organization of the mouse Ikaros gene. The entire gene is 80-90 kB in length. Intronic or uncharacterized DNA is indicated as a line between 5' and 3'. Exons are indicated as boxes. Lines numbered f2, f10, f4, and f8 indicate phage inserts corresponding to the sequence immediately above. Restriction sites are indicated by the usual abbreviations.

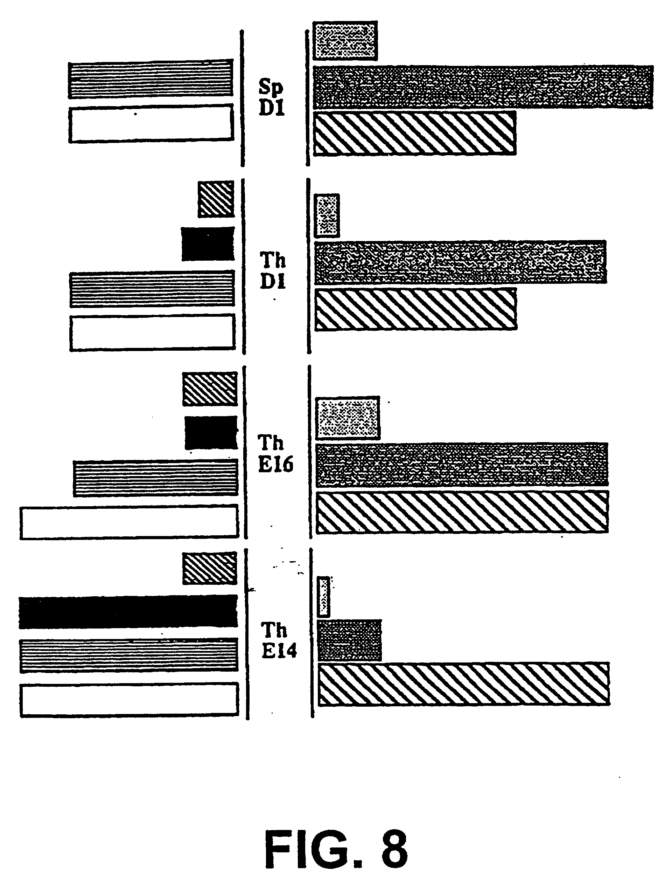

Fig. 8 is a model of Ikaros isoform control of differential gene expressions. Th=thymus; Sp=spleen; Ex=day of embryonic development; Dx = day of postnatal life. The left hand column represents the relative expression of an isoform at a given developmental stage. Open bar=Ik-1; Horizontal stripes=Ik-2; Diagonal stripes=Ik-3; and solid bar=Ik-4. The right hand side shows the resulting reactivity of Ikaros binding sites at a given developmental stage. Light bars=low affinity sites (sites at which isoforms 1, 2, 3 and 4 bind with similar affinities); Dark bars=high affinity inverted or direct repeat containing sites (e.g., NFKB sites, Ik1-4 bind with high affinity); Diagonal bars=single high affinity sites (sites where Ik1 and Ik2 bind but Ik3 and Ik4 don't bind (and therefore won't attenuate the binding of Ik-1 and Ik-2).

### Ikaros: A master regulator of hemopoietic differentiation

A hemopoietic stem cell in the appropriate microenvironment will commit and differentiate into one of many cell linages. Signal transduction molecules and transcription factors operating at distinct check points in this developmental pathway will specify the cell fate of these early progenitors. Such molecules are viewed as master regulators in development but also serve as markers for the ill defined stages of early hemopoiesis.

Studies on the transcriptional mechanisms that underlie gene expression in T and B cells have identified several transcriptional factors involved in lymphocyte differentiation. However, some of these genes appear to play a role in several developmental systems as determined by their non restricted pattern of expression in the adult and in the developing embryo. The HMG box DNA binding proteins TCF and LEF restricted to T cells and early lymphocytes in the adult are widely expressed in the developing embryo. The T cell specific GATA-3 transcription factor is also expressed outside the hemopoietic system in the early embryo. The ets family members Ets-1 and Elf-1 are widely distributed as well. In addition, the binding affinity and transcription potential of most of these proteins is controlled by other tissue restricted molecules. The ets proteins interact with additional factors for high affinity binding to their cognate sequences. TCFI, LEF and ets-1 must interact with other lymphoid restricted accessory proteins to activate transcription.

In search of a lymphoid restricted transcriptional enhancer, in control of gene expression in early T cells, we have isolated the Ikaros gene, which encodes a zinc finger DNA binding protein. In the early embryo, the Ikaros gene is expressed in the hemopoietic liver but from mid to late gestation becomes restricted to the thymus. The only other embryonic site with Ikaros mRNA is a small area in the corpus striatum. In the adult, the Ikaros mRNA is detected only in the thymus and in the spleen (Georgopoulos *et al.* 1992). The Ikaros gene functions as a transcriptional enhancer when ectopically expressed in non lymphoid cells.

The Ikaros gene plays an important role in early lymphocyte and T cell differentiation. The Ikaros gene is abundantly expressed at early embryonic hemopoietic sites is later on restricted in the developing thymus. The thymus together with the spleen are the prime sites of expression in the adult. This highly enriched expression of the Ikaros gene was also found in early and mature primary T cells and cell lines. This restricted pattern of expression of the Ikaros gene at sites where embryonic and adult T cell progenitors originate together with the ability of the encoded protein to activate transcription from the regulatory domain of an early T cell differentiation antigen supported a determining role in T cell specification.

Differential splicing at the Ikaros genomic locus generates at least five transcripts that encode proteins with distinct DNA binding domains. These Ikaros protein isoforms (IK-1, IK-2, IK-3, IK-4, IK-5) have overlapping but also distinct DNA binding specificity dictated by the differential usage of zinc finger modules at their N-terminus. The core binding site for four of the Ikaros proteins is the GGGA motif but outside this sequence their specificity differs dramatically. The IK-3 protein shows strong preferences for bases at both the 5' and 3' flanking sequences which restricts the number of sites it can bind to. The Ik-1 protein also exhibits strong preference for some of these flanking bases and can bind to wider range of sequences. The Ik-2 protein, the most promiscuous of the three proteins, can bind to sites with just the GGGAa/t motif. Finally, the Ik-4 protein with similar sequences specificity to Ik-1 binds with high affinity only when a second site is in close proximity suggesting cooperative site occupancy by this protein. A number of putative binding sites for the Ikaros proteins were identified in the enhancers of cell T cell receptor -δ -β and -α and the CD3- δ, -ε and -γ genes, in the HIV LTR in the IL2-R promoter and in a variety of other lymphocyte restricted genes. One of these sites, the NFkB variant in the I1-2Rα enhancer, binds all of these proteins with high affinity. This NFkB motif, a crucial regulatory element for expression of the I12 Receptor α in T cells is a strongly activated by the Ik-1 and Ik-2 isoforms in T and non T cells. The IL-2Rα gene is expressed at high levels in activated T cells but also in fetal thymocytes which also express high levels of the Ikaros gene. Thus, gene regulation of at least the IL2α Receptor during T cell differentiation and activation may be controlled by the intricate interplay of NFkB and Ikaros transcription factors interacting on common grounds.

The embryonic expression pattern and activation potential of the Ikaros isoform is markedly different. The strong transcription factors Ik-1 and Ik-2 are abundantly expressed in the early fetal liver, in the maturing thymus and in a small area of the developing brain while the weak activators Ik-3, Ik-4 are expressed at lower levels. However, since the Ik-1 and particularly the Ik-2 proteins bind a wider range of sites than Ik-3 and Ik-4, the available molecules that bind to the same sequences as Ik-3 and Ik-4 may be at similar concentration. Moreover, in the day 14 embryonic thymus the weak activator Ik-4 is present at equal or greater levels than Ik-1 and Ik-2 isoforms. Competition between Ik-4, Ik-1 and Ik-3 for perfect or imperfect inverted and direct repeats of their recognition motif (NFkB variants) may mediate low levels of transcriptional activation from these sites in the early thymus. Steady state levels of the activating Ikaros factors in combination to the decreasing levels of the non activating Ik-4 protein maturing thymocytes may turn on the activity of these double sites in the developing thymus. This may result in denovo expression of stage specific T cell differentiation antigens. Finally, activation from low affinity binding sites may only be possible in these late stages of T cell differentiation when the Ikaros activating proteins are in excess. Concentration gradients of the Drosphila NKkB homologue Dorsal and distinct protein-protein interactions with other nuclear factors is responsible for the activation level and threshold response from low and high affinity binding sites in the fly embryo. Fig. 8 provides a model in which the relative concentrations of Ikaros isoforms at different developmental stages confer different reactivites on the various sites.

The transcriptional activity of the Ik-3 and Ik-4 proteins may be further regulated by T cell restricted signals mediating postranslational modifications or by protein -protein interactions. The Ik-4 protein binds NFkB motif in a cooperative fashion and may therefore interact in situ with other members of the Ikaros or of the NFkB family. These protein-protein-DNA complexes may dictate a differential transcriptional outcome.

The differential expression of the Ikaros isoforms during T cell ontogeny, their overlapping but distinct binding specificity and their diverse transcription potential may be responsible for the orderly activation of stage specific T cell differentiation markers. Multiple layers of gene expression in the developing lymphocyte can be amenable to regulation by these proteins. Synergistic interactions and/or competition between the members of the Ikaros family and other factors in these cells on qualitatively similar and distinct target sites could dictate the genetic make up of the resting and activated lymphocyte. This functional dissection of the Ikaros gene strongly support its proposed function as a master gene in lymphocytes. The role of the Ikaros gene as the necessary genetic switch for early hemopoiesis and T cell development in ultimately being addressed in gene ablation studies in transgenic animals.

### Mutational Analysis of the δ Element of the CD3δ Enhancer

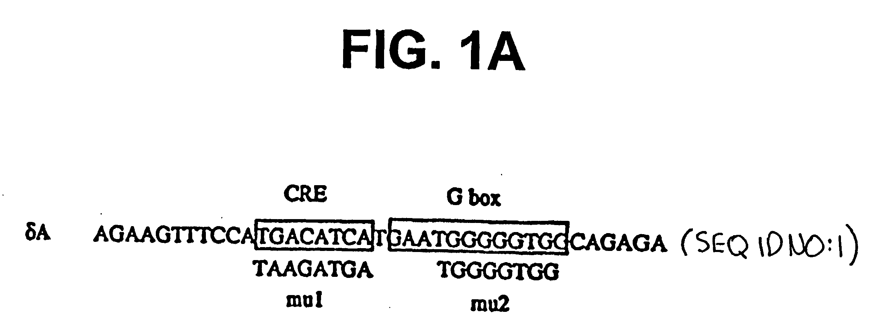

One approach useful for characterizing early events in T cell differentiation is to study the regulation of transcription of T cell restricted antigens. We have chosen the transcriptional control of one of the earliest and definitive T cell differentiation markers, the CD3δ gene of the CD3/TCR complex. In order to identify a transcription factor expressed at or earlier than T cell commitment which can function as a genetic switch regulating entry into the T cell lineage we have characterized a T cell specific enhancer mediating expression of this gene. This enhancer is comprised of two functionally distinct elements δA and δB with activity restricted in T cells. Mutational analysis of the δA element has further identified two transcriptionally active binding sites, a CRE (Cyclic AMP response)- like element and a G rich sequence motif both of which are required for full activity of the δA element and the CD3 enhancer, see Fig. 1.

Fig. 1 depicts the functional dissection of the δA element of the CD3δ enhancer. Fig. 1A shows the binding sites in the δ element (SEQ ID NO:1). The boxed sequences represent the CRE-like and the G rich motif both important for activity of the δA element. Mutations introduced in the δA element are shown below the sequence.

Fig. 1B shows the contribution of the CRE and the G box to the activity of the δ A element and the CD3δ enhancer as analyzed by transient expression assays in the T cell line EL4. The activity of the tkCAT reporter gene under the control of wild type δ A, δAmul and δAmu2 as reiterated elements or in the context of the CD3δ enhancer was determined as described in Georgopoulos et al. (1992) Mol . Cell . Biol. 12:747. Reporter gene activation (R.A.) was expressed as the ratio of Chloramphenicol Acetyl Transferase (CAT) to Growth Hormone (GH) activity estimated for each transfection assay. Fig. 1C shows that the expression of the Ikaros gene (Ik-2)in non T cells upregulates the activity of the δA element. The CDM8 and CDM8:Ikaros recombinant I expression vectors were cotransfected with the tkcat 3δA, tkcat 3δ Amul, tkcat 3δAmu2 and tkcat δenhancer reporter genes in CV1 (kidney epithelial) cells as described in Georgopoulos et al. (1992). The ratio of reporter activation (R.A.=CAT/GH) in the presence and absence of Ikaros expression was estimated. Three isoforms of the ubiquitously expressed CRE-Binding Protein were cloned from T cells for their ability to interact with the CRE-like binding site of the δA element, see Georgopoulos et al. (1992). Although dominant negative mutants of this protein down regulate the activity of this enhancer element in T cells, expression of this transcription factor in all hemopoietic and non hemopoietic cells argues against it being the switch that activates the CD3δ enhancer in the early prothymocyte progenitor. A variant of the δA element (δAmul-CRE) was used to screen a T cell expression library as described in Georgopoulos et al. (1992). As described below, a T cell restricted cDNA was cloned encoding for a novel zinc finger protein (Ikaros) that binds to the G box of the A element.

### Cloning the Ikaros Gene

A T cell expression cDNA library from the mature T cell line EL4 was constructed into the A ZAP phage vector.

A multimerized oligonucleotide encoding sequence (SEQ ID NO:4) from one of the protein binding sites of the CD38 enhancer was used as a radiolabelled probe to screen this expression library for the T cell specific proteins that bind and mediate enhancer function by the southwestern protocol of Singh and McKnight. Four gene encoding DNA binding proteins were isolated. One, the Ikaros gene, encoded a T cell specific protein.

### The Sequence of Ikaros

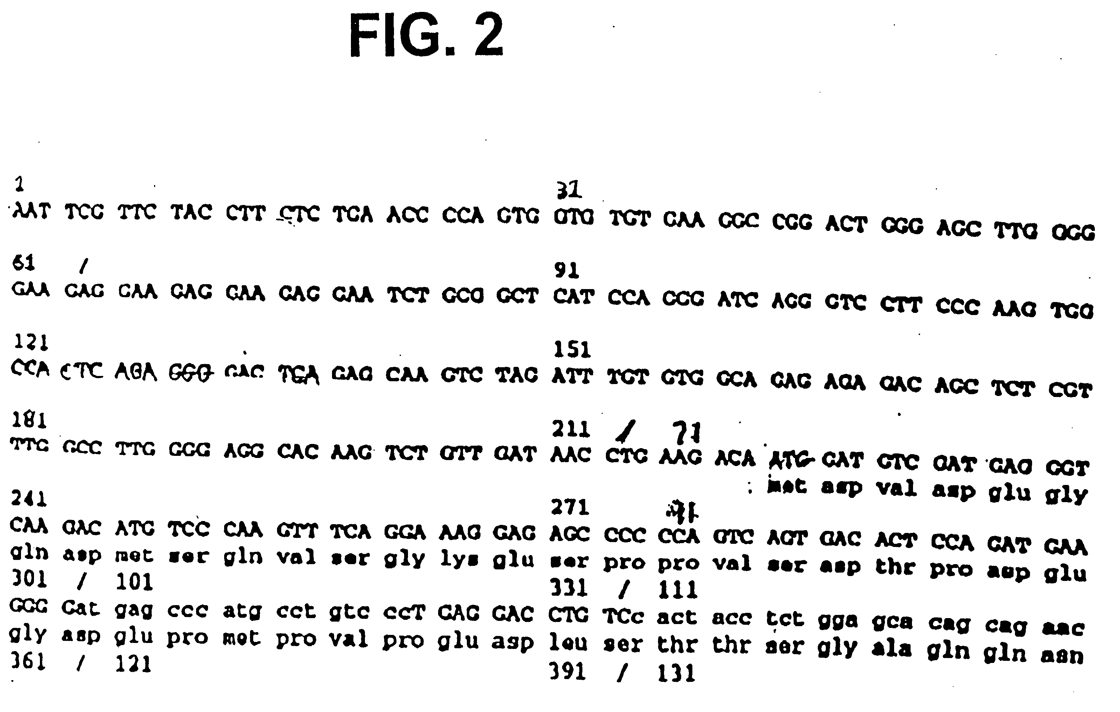

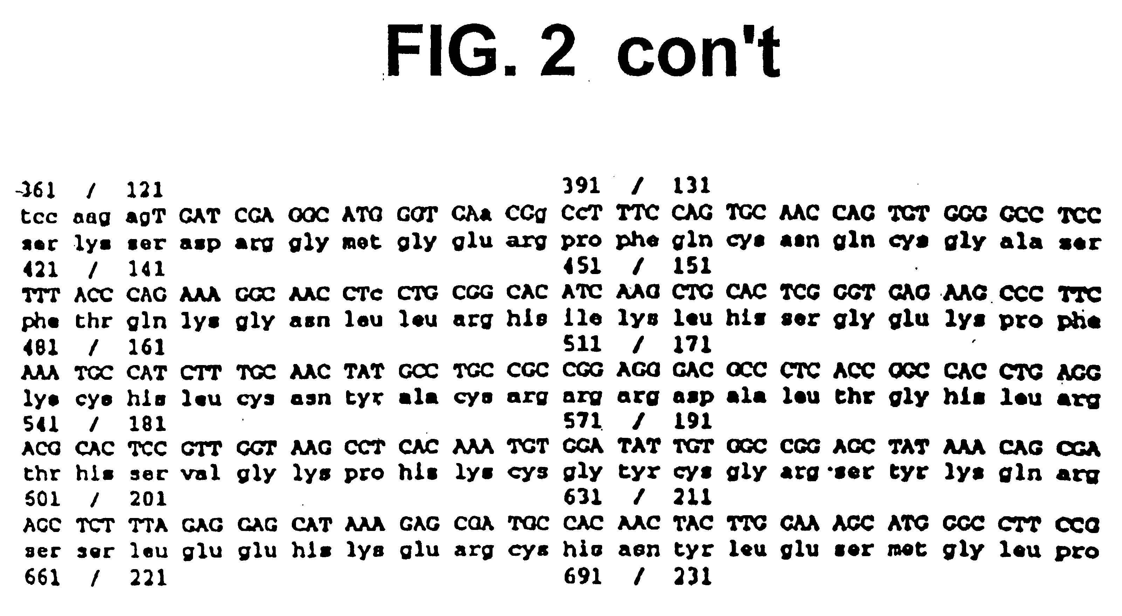

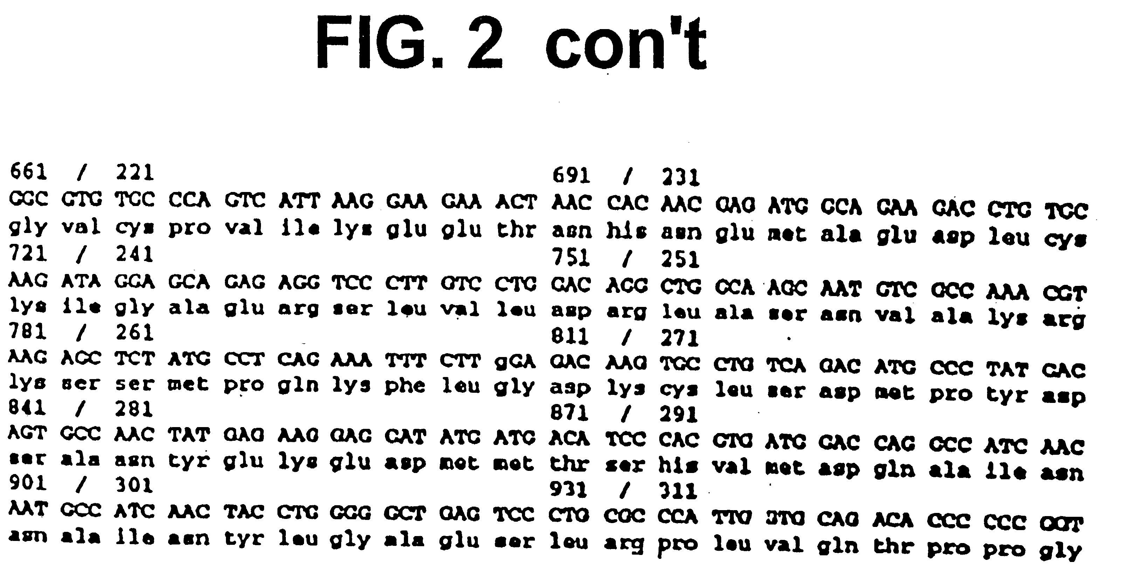

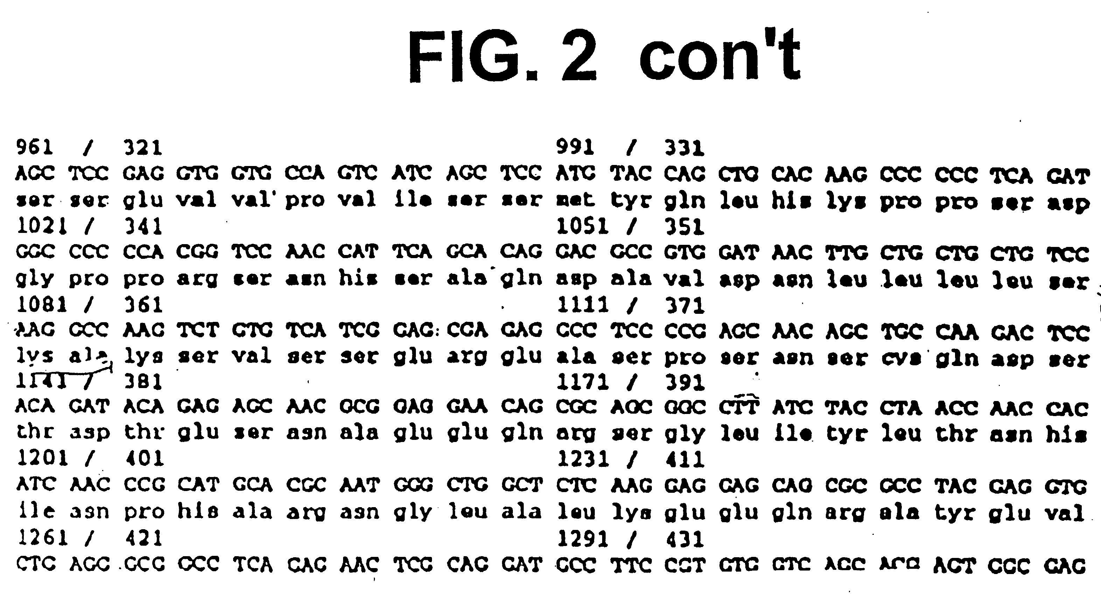

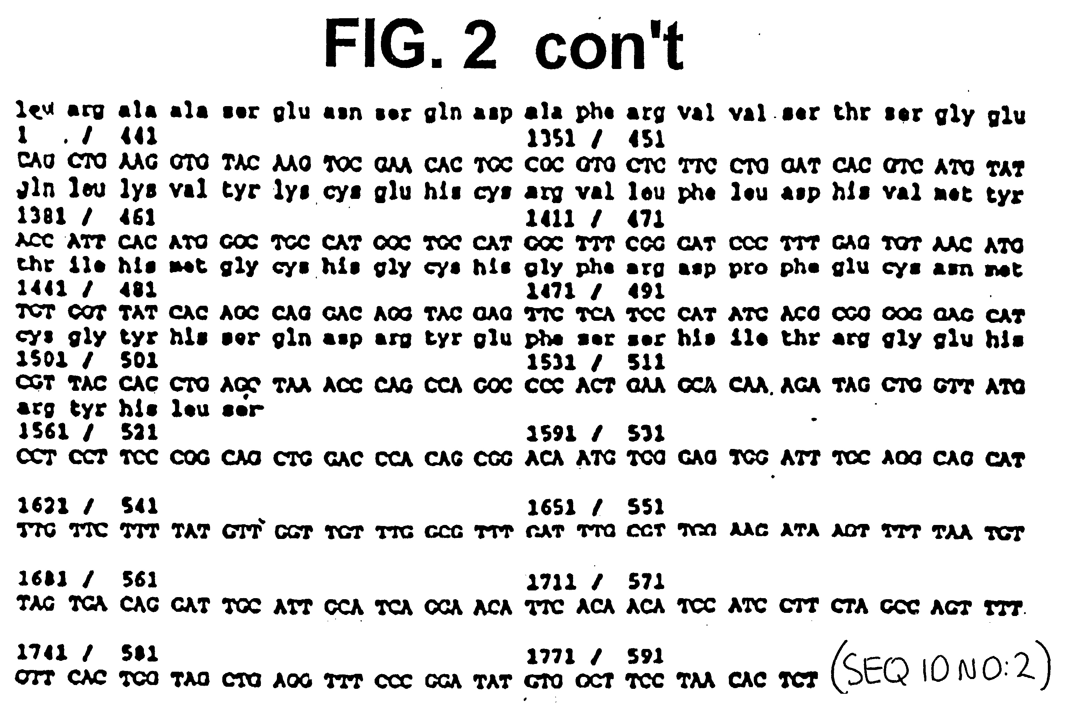

The sequence of the Ikaros gene was determined using the Sanger dideoxyl sequencing protocol. The derived amino acid sequence was determined using the MAP program of GCG (available from the University of Wisconsin) and Strider sequence analysis programs. Fig. 2 provides the sequence of an Ikaros cDNA and the derived amino acid sequence encoded thereby (SEQ ID NO:2).

### An Ikaros Protein

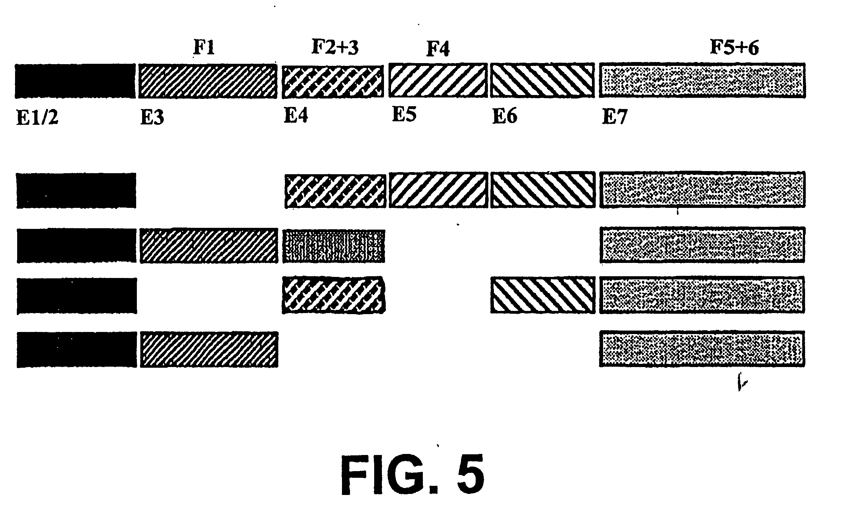

The Ikaros protein shown in Fig. 2 (Ik-2) is comprised of 431 amino acids with five CX₂CX₁₂HX₃H zinc finger motifs organized in two separate clusters. (See also Fig. 5.) The first cluster of three fingers is located 59 amino acids from the initiating methionine, while the second cluster is found at the C terminus of the protein 245 amino acids downstream from the first. Two of the finger modules of this protein deviate from the consensus amino acid composition of the Cys-His family of zinc fingers; finger 3 in the first cluster and finger 5 at the C terminus have four amino acids between the histidine residues. This arrangement of zinc fingers in two widely separated regions is reminiscent of that of the Drosophila segmentation gap gene Hunchback. Similarity searches in the protein data base revealed a 43% identity between the second finger cluster of Ikaros and Hunchback at the C terminus of these molecules. This similarity at the C terminus of these proteins and the similar arrangement of their finger domains raises the possibility that these proteins are evolutionary related and belong to a subfamily of zinc finger proteins conserved across species.

### Ikaros isoforms

In addition to the cDNA corresponding to Ik-2, four other cDNAs produced by differential splicing at the Ikaros genomic locus were cloned. These isoform encoding cDNAs were identified using a 300 bp fragment from the 3' of the previously characterized Ikaros cDNA (Ik-2, Fig. 1). As shown in Fig. 4 and 5, each isoform is derived from three or more of six exons, referred to as E1/2, E3, E4, E5, E6 and E7. All five cDNAs share exons E1/2 and E7 encoding respectively for the N-53 and C-terminal 236 amino acid domains. These five cDNAs consist of different combinations of exons E3-6 encoding the N-terminal zinc finger domain. The Ik-1 cDNA encodes a 58 kD protein with four zinc fingers at its N-terminus and two at its C-terminus and has the strongest similarity to the Drosophila segmentation protein Hunchback (Zinc fingers are indicated as F1, F2+F3, F4, and F5+F6 in Fig. 5). The Ik-2 and Ik-3 cDNAs encode 48kd proteins with overlapping but different combinations of zinc fingers. The IK-3 contains fingers 1,2,3 while Ik-2 contains fingers 2, 3 and 4. The 43.5 kD Ik-4 protein has two fingers at its N-terminus also present in Ik-1 and Ik-2. The Ik-5 cDNA encodes a 42kd protein with only one N-terminal finger shared by Ik-1 and Ik-3 (Fig. 1). This differential usage of the zinc finger modules by the Ikaros proteins support an overlapping but differential DNA binding specificity.

cDNA cloning of isoforms was performed as follows. A cDNA library made from the T cell line EL4 in λZAP was screened at high stringency with a 300 bp fragment from the 3' of the previously described Ikaros cDNA (isoform2). Positive clones were characterized by sequencing using an antisense primer from the 5' of exon 7.

### Ikaros Expression

### Tissue Specific Expression of the Ikaros Gene

The Ikaros gene is expressed in T cells and their progeny. In the adult mouse, Ikaros mRNA is restricted to the thymus and the spleen with expression in the thymus being about 3 fold higher than the spleen. Spleen cells preparations depleted of T cells expressed very low levels of this message. Examination of Ikaros expression in cell lines confirm the view that the Ikaros gene is expressed in T cells and their progeny. Ikaros mRNA was detected in a number of T lymphoma cell lines. The T cell line EL4 expressed the highest levels while DO11.10, BW5147 and SL12.1 lymphomas showed moderate to low expression. No expression or very low levels were detected in cell lines representing other hemopoietic lineages including the bone marrow derived progenitor cells FDCP1 that exhibit myeloid morphology and differentiation potential, the mast cell line RBL, the macrophage line J774 (detected expression is 25 fold lower than that in thymocytes) and MEL cells which were induced to differentiate into erythroid cells. Nevertheless, moderate levels of Ikaros mRNA were detected in the B cell lymphoma A20 and in the proerythroleukemia cell line MEL. Immortalization of these cell lines and their leukemic phenotype may account for aberrant expression of this nuclear factor which does not appear to be expressed at significant levels in normal B cells (spleen T cell depleted population, or in erythroid progenitors in vivo (from in situ data). Alternatively expression of this thymocyte restricted factor in these cell lines may reflect the existence of an early progenitor with the ability to differentiate into the lymphoid or the erythroid lineage.

Tissue distribution of the Ikaros gene was determined by Northern hybridization of total RNAs prepared from: the T lymphoma cell lines EL4, BW5147, DO11.10, SL12.1; the B cell lymphoma A 20; the tissues of thymus, spleen, kidney, brain and heart isolated from an adult mouse; spleen thymocytes (total and polyA-RNA); bone marrow derived stem cell progenitors FDCP1; macrophage cell line J774; mast cell line RBL; undifferentiated MEL and 58hr DMSO induced MEL cells; and finally T depleted spleen cells (TDSC). A 320bp fragment (bp 1230-1550) from the 3' end of the Ikaros Ik-2 cDNA was used as a probe.

### Temporal Regulation of the Expression of the Ikaros Gene

To determine when in hemopoiesis the Ikaros gene becomes activated its expression was studied in situ in the developing mouse embryo. Hemopoiesis begins at day seven in the yolk sac of the mouse embryo with the generation of a large population of primitive erythroblasts. The Ikaros mRNA is not detected in the yolk sac at day 8 in contrast to the erythroid specific transcription factor GATA-1 which is expressed at this time in development. In the embryo proper, expression of Ikaros is first detected in the early liver rudiment at the onset of its hemopoietic function (day-9 1/2 - 10 1/2). At this time, pluripotent stem cells as well as more restricted progenitors are found in the liver which can successfully reconstitute irradiated animals with the whole spectrum of hemopoietic lineages. Expression of the Ikaros gene remains strong in the liver up to day fourteen and begins to decline thereafter although the liver is the major site of hemopoiesis through mid gestation and remains active through birth. The declining expression of the Ikaros gene in the fetal liver at mid gestation is consistent with changes in the hemopoietic profiles from pluripotent stem cells to more committed erythroid progenitors.

The second site of Ikaros expression is in the thymic rudiment around day 12 when lymphopoietic stem cells are first colonizing this organ. A group of expressing cells is detected at the center of the thymic rudiment surrounded by non expressing cells in the periphery. Expression in the developing thymus becomes quite prominent by day 16 and persists throughout embryogenesis to the adult organism. At these developmental stages expression of Ikaros mRNA is detected throughout the thymus with levels in the medulla sections being slightly more elevated than these in the cortex.

Ikaros expression is first detected in the spleen during late gestation at low levels compared to those of the thymus (day 19). Although the spleen is active in erythropoiesis and myelopoiesis from mid-gestation, its population with mature T cells from the thymus takes place late in embryogenesis and correlates with the late expression of the Ikaros gene. No expression of Ikaros message is detected in the bone marrow of the long bones or the spinal column at day 19 in contrast to the myeloid specific factor Spyl and to the erythroid factor GATA-1. The pattern of expression of the Ikaros gene detected in distinct hemopoietic sites throughout embryonic development is consistent with its restriction to T cells and their progenitors. The only other site in the mouse embryo that exhibited Ikaros expression was a restricted area in the brain which gives rise to the proximal corpus striatum (day 12 through 19).

Embryos were harvested from time pregnant CD1 mice (Charles River) and were fixed in 4% paraformaldehyde for 2 hours to 2 days depending on size. A series of dehydration steps was performed in alcohols followed by xylenes before paraplast embedding. Sections were prepared and treated according to published protocols. Sense and antisense P-UTP RNA probes 300 bp in size were made from the 3' untranslated region of the Ikaros cDNA and were used to hybridize to selected slides at 48°C overnight. After high stringency washings slides were dehydrated and dipped in diluted photographic emulsion (NBT2) for 3 weeks. Dipped slides were developed, stained with Giemsa and analyzed by bright and dark field microscopy.

### Expression of Ikaros Isoforms

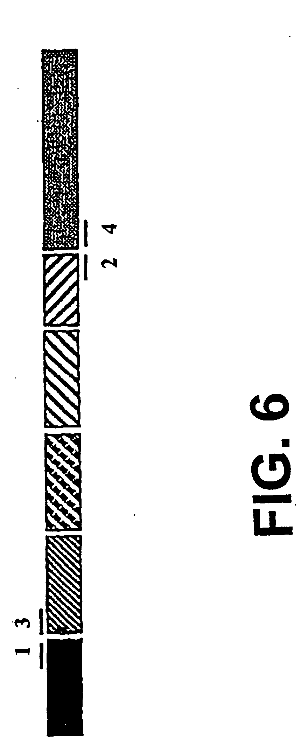

The pattern of Ikaros isoforms expression in the developing embryo was studied. Two sets of primers were used to amplify the five cDNAs as distinct sized bands from embryonic and postnatal tissues(Fig. 6). A third set of primers complementary to the β-actin cDNA was used to normalize the amount of cDNA used n the reaction. Primers 1/2 amplified a 720, a 457 and a 335 bp fragment from the Ik-1, Ik-2 and Ik-4 cDNAs. Primers 3/4 amplified a 715, a 458 and a 293 bp fragment from the Ik-1, Ik-3 and Ik-5 cDNAs. A 650bp band detected is an artifact of Ik-1 and Ik-2 coamplification representing Ik-1/Ik-2 and Ik-1/Ik-3 hybrid molecules. It is present at significant levels at the later amplification cycles when the primers to Ik-1, Ik-2 and Ik-3 ratio is decreased. This band is also detected when we coamplify Ik-1, Ik-2 and Ik-3 DNA templates. The identity of the above described bands were also confirmed by cloning and sequencing. It is noteworthy that the 650 bp species was never cloned as a novel of cDNA.

During embryonic development all five Ikaros mRNAs were expressed in hemopoietic centers and in the brain at relatively different levels. The Ik-1 mRNA was abundantly expressed in the early fetal liver and in the maturing thymus while Ik-2 was second in the relative concentration. The Ik-4 isoform was expressed at low levels compared to Ik-1 and Ik-2 in the early fetal liver and in the maturing thymus (liver E14, thymus E16 and D1). However it was expressed at comparable amounts to Ik-1 and Ik-2 in the early thymus and mid-gestation liver (Table 1, thymus E14, liver E16). The Ik-3 and Ik-5 isoforms were expressed but at significantly lower levels than Ik-1 and Ik-2 throughout development (Table 1). All five isoforms were expressed in the embryonic brain. The Ik-1 was the most abundant mRNA, Ik-2 and Ik-4 were present at similar but lower levels while Ik-3 and Ik-5 were the lease expressed.

The expression pattern of the Ikaros isoforms detected in the late embryonic thymus persisted past birth while the declining liver expression was switched off. The neonatal spleen expressed only Ik-1 and Ik-2 mRNAs at significant amounts. Low concentration of Ik-1 were still detected in the neonatal brain. These data agree and further supplement our previous in situ hybridization studies performed using an RNA probe made from the 3' of the Ikaros gene shared by all identified Ikaros splicing products.

**Table 1.**

| A summary of the embryonic expression patterns for the Ik-1-5 transcripts. | | | | | | |
|---|---|---|---|---|---|---|
| | | **Ik-1** | **Ik-2** | **Ik-3** | **Ik-4** | **Ik-5** |
| **Liver** | **E14** | +++++ | ++++ | ++ | ++/- | - |
| | **E16** | +++ | ++ | +/- | ++/- | - |
| | **D1** | + | - | - | - | - |
| **Thymus** | **E14** | +++ | +++ | +/- | +++ | - |
| | **E16** | *++++* | +++ | +/- | + | +/- |
| | **D1** | +++ | +++ | - | + | + |
| **Brain** | **E14** | ++ | + | +/- | + | + |
| | **E16** | ++ | + | +/- | + | + |
| | **D1** | + | - | - | - | - |
| **Spleen** | **D1** | +++ | +++ | - | - | - |

Embryonic tissues were obtained from embryos of time pregnant mothers. 2µ gs of total RNA prepared from the thymus, liver, brain and spleen at different stages of embryonic development were used for cDNA synthesis with random hexamers and Superscript RNaseH. 1/10th of cDNA made was used in PCR amplification with the 1/20, 3/4 and actin A/B set of primers. PCR reactions were denatured at 95°C for 5 minutes, polymerase was added at 80°C, and then were amplified for 25 cycles at 94° C for 45", 63°C for 1' and 72°C for 1'. PCR amplification for the actin cDNAs were performed for 30 cycles. Products were separated on 2% Seakam FMC agarose, bands were excised, cloned (TA cloning kit, Clonteck) and sequenced to verify their identity.

### Ikaros stimulates the transcription from the δA element

### Initial Transcriptional Studies

We examined whether the Ikaros protein that can bind to the δA element can also activate transcription from this binding site. The tkCAT reporter gene under the control of either a reiterated δA binding site (+/-CRE/-G) or under the control of the CD3δ enhancer was cotransfected with a recombinant vector expressing the Ikaros gene in the kidney epithelial cell line CV1. Expression of the Ikaros gene in non T cells strongly stimulated transcription from the G box of a reiterated δA element and in the context of the CD3δ enhancer (see Fig. 1C). Activity of the δA and δAmu1(-CRE) elements was stimulated by eight and seven fold respectively while expression of the CD3δ enhancer was stimulated by five fold. Since the CD3δ enhancer is comprised of at least two regulatory elements, expression of all the transcription factors that bind to these sites is necessary for its full activation potential. Expression of the Ikaros gene did not significantly stimulate the activity of the thymidine kinase promoter or of the δAmu2(-Gbox) element (see Fig. 1C). These data confirms our hypothesis that the Ikaros gene can control activity of the T cell specific δA element of the CD3δ enhancer and suggests that it can mediate expression of at least the CD3δ gene in T cells.

The expression pattern of the Ikaros protein, and its ability to modulate the activity of the CD3δ enhancer, is consistent with a role in mediating gene expression in T cells in the embryo and in the adult. Its early expression in fetal liver hemopoietic stem cells suggests that it may be expressed in early prothymocyte progenitors and raiser the possibility that it is responsible for commitment of a pluripotent stem cell to the T cell lineage.

### Binding Site Selections for the Ikaros 1-3 Isoforms

To investigate the possibility that differential usage of zing finger modules at the N-terminus of the five Ikaros isoforms contributes to their DNA binding a specificity we cloned high affinity binding sites for three of these proteins. The Ik-1, Ik-2 and Ik-3 proteins were selected since they contain either all four (Ik-1) or two distinct combination of three (Ik-2 and Ik-3) from the pool of the N-terminal four fingers (Fig. 5). We expected these proteins to overlap in specificity with Ik-4 and Ik-5 proteins which contain only two or one of these putative DNA binding modules.

After five rounds of binding site selections from a pool of random oligonucleotides the Ik-1-, Ik-2- and Ik-3- selected oligomers were cloned, sequenced and aligned to a shared motif (Tables 2, 3 and 4 in the tables, bold face type indicates conserved sequence).

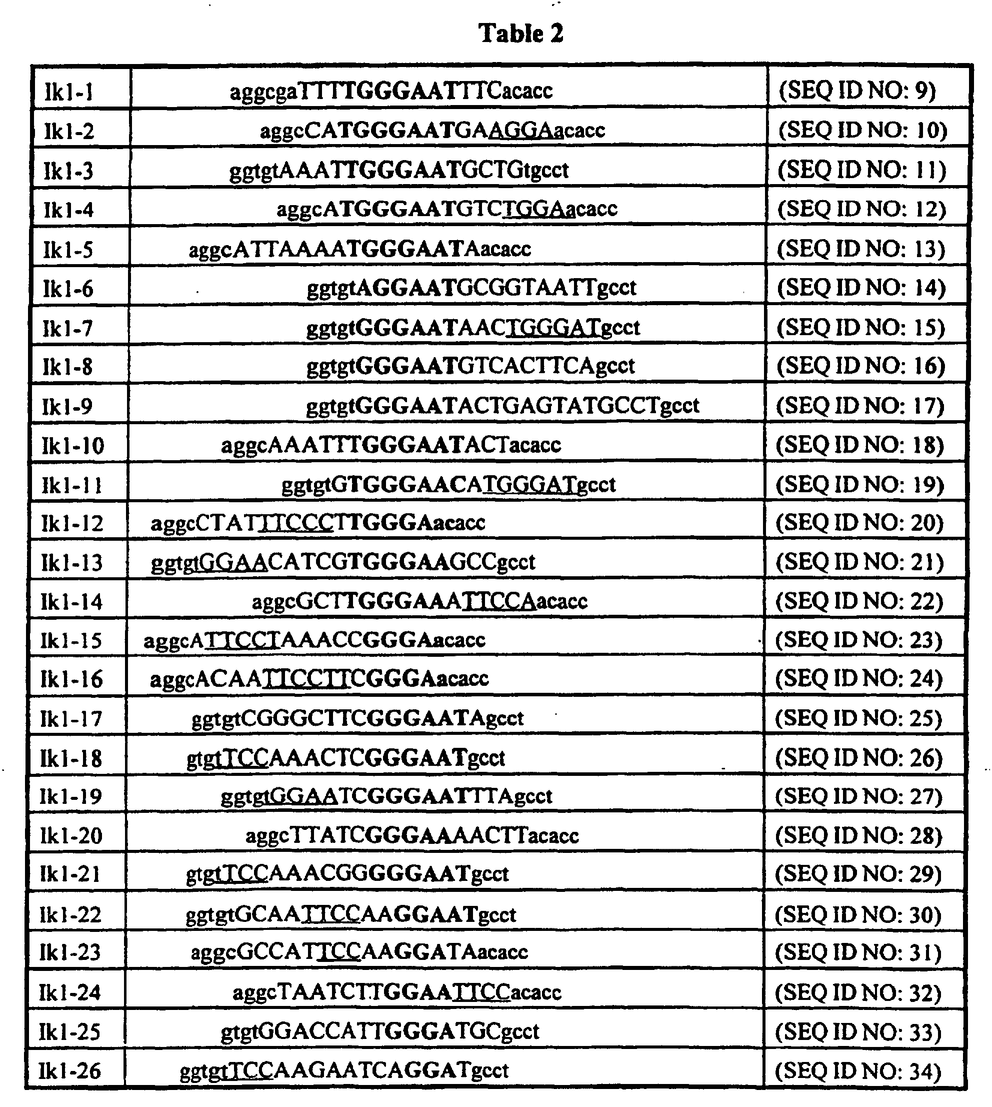

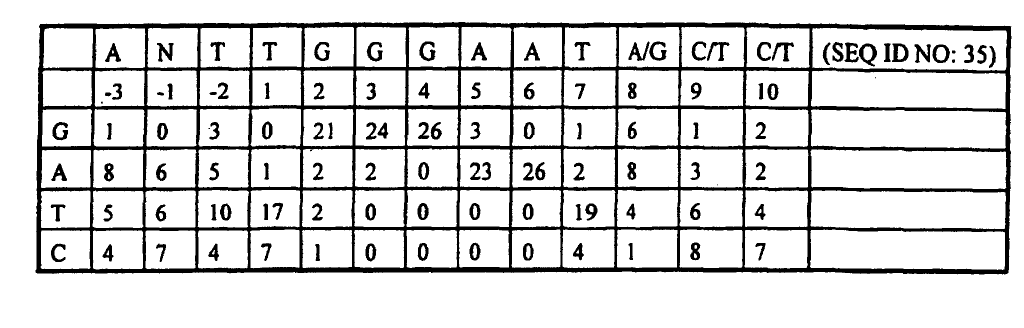

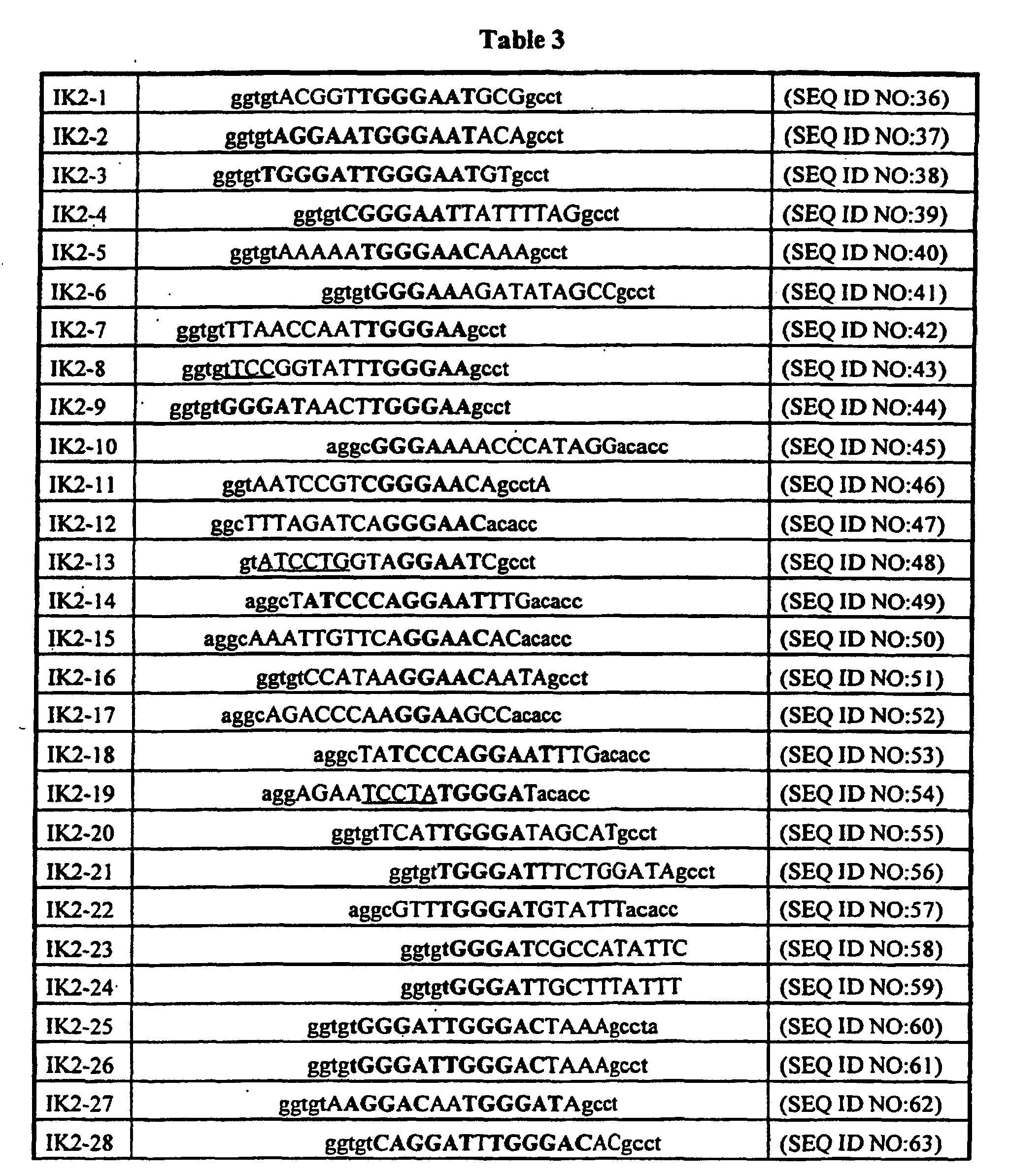

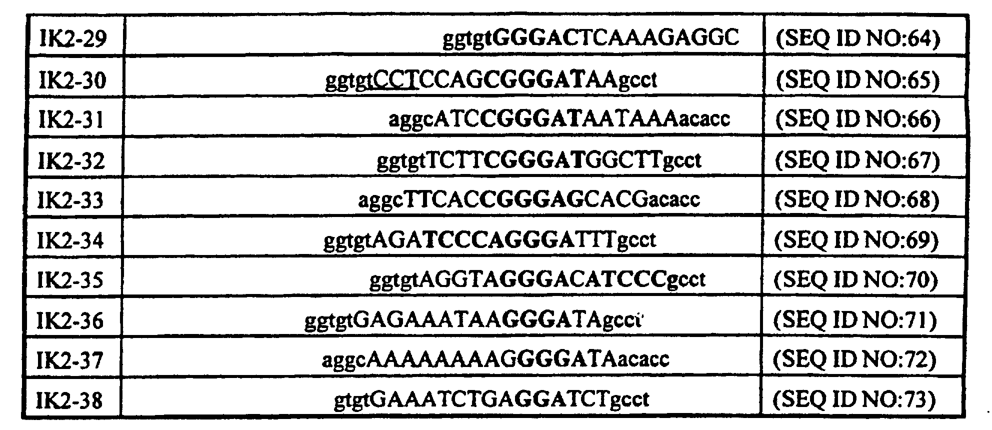

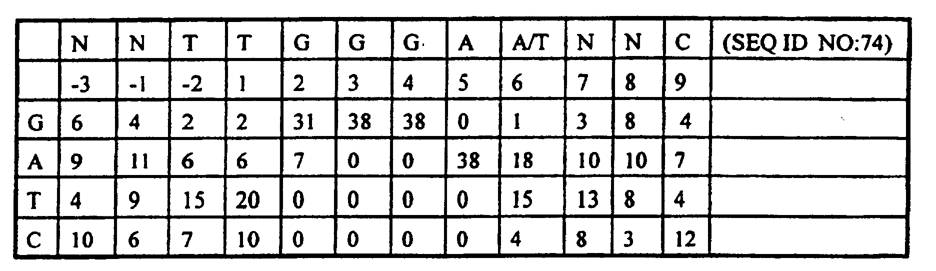

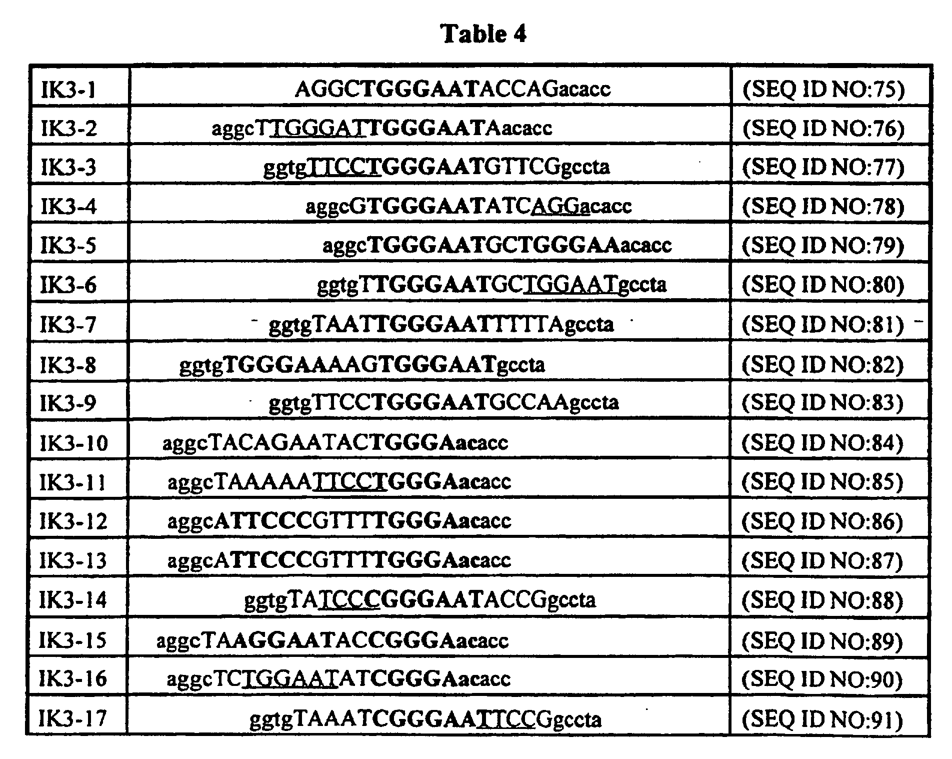

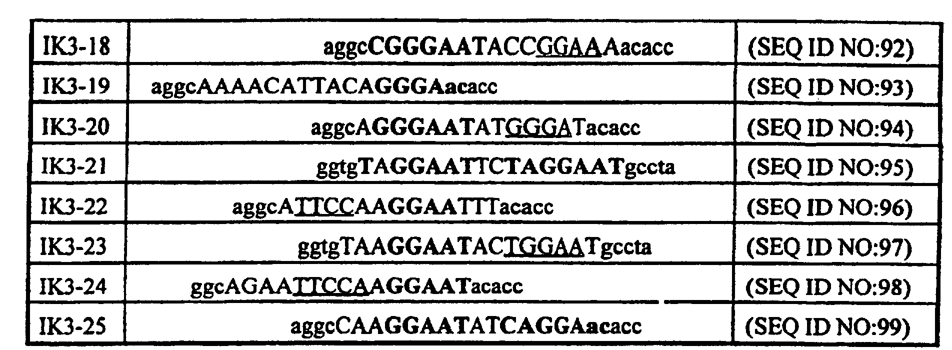

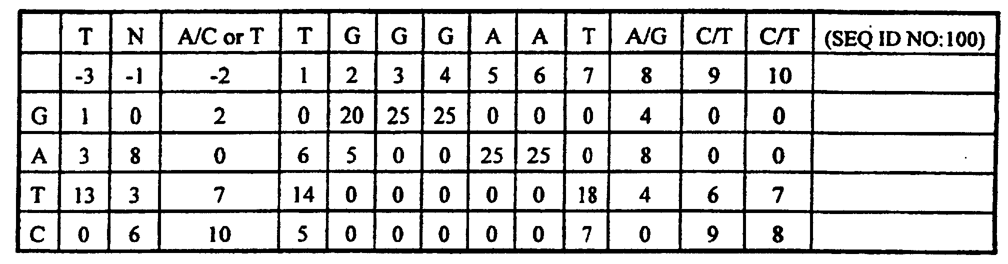

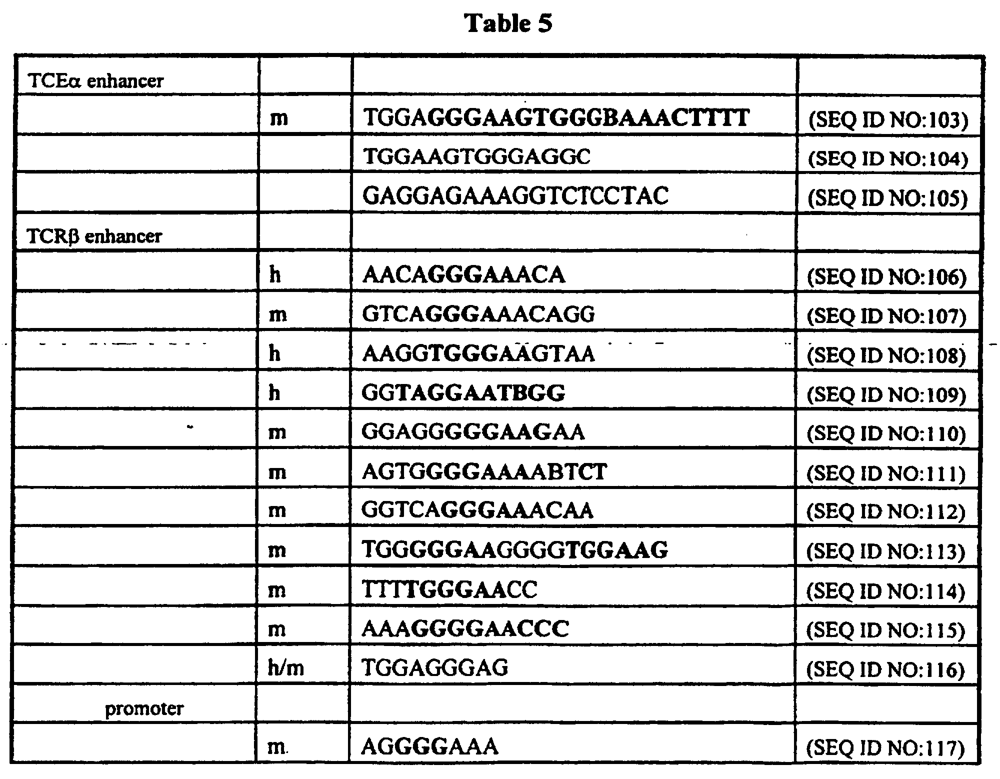

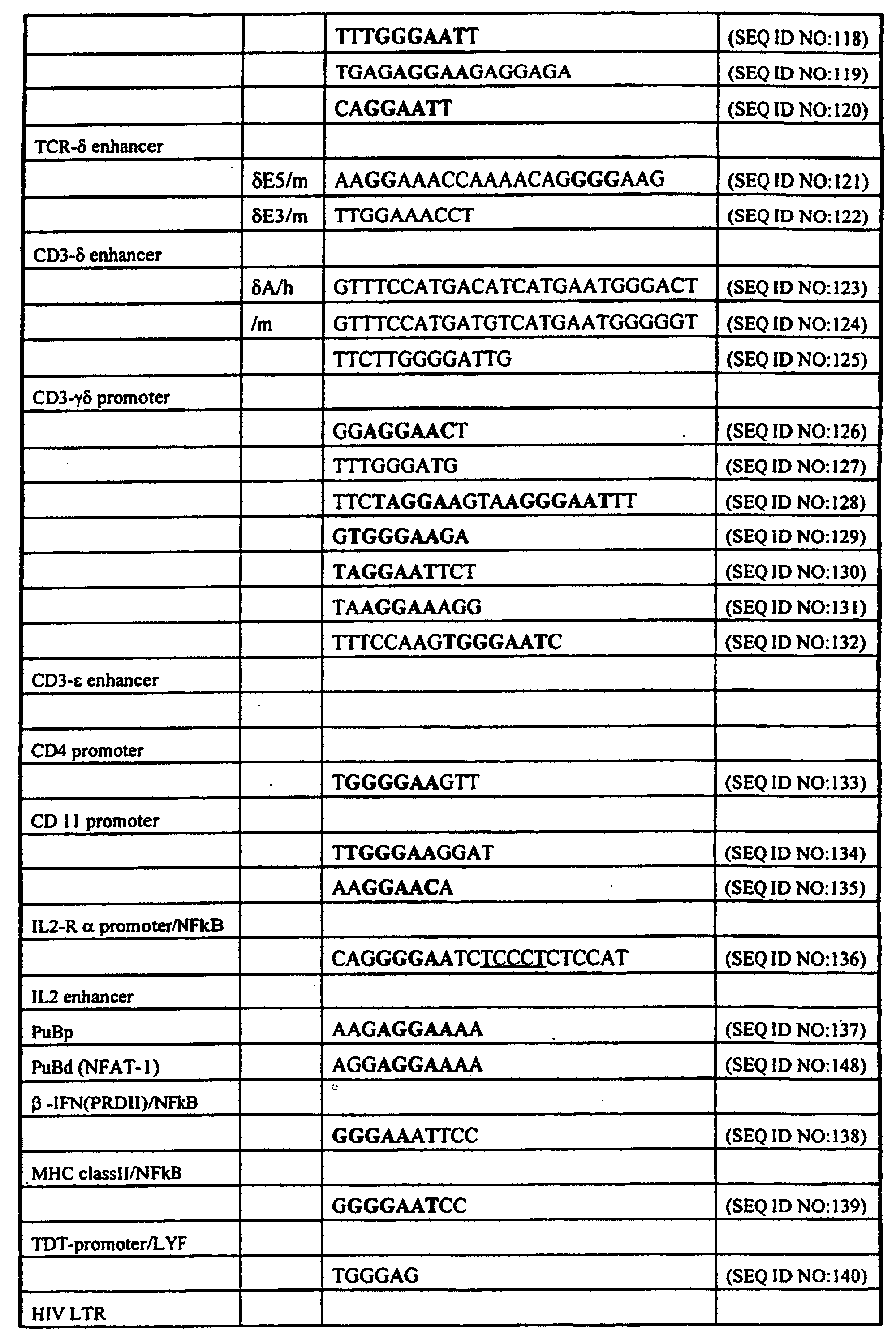

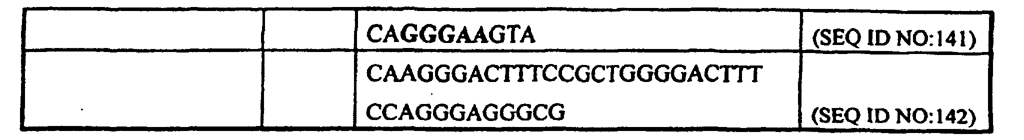

A consensus recognition sequence for each of these proteins was derived.
The Ik-1, Ik-2 and Ik-3 core motifs were , respectively;

The Ik-1 and Ik-3 sequences shared the seven base pair core **T-G-G-G-A-A-T** (SEQ ID NO: 149). The Ik-3 protein showed strong reference for particular nucleotides both at the 5' and 3' flanking positions of this motif while the Ik-1 protein did not select for any particular bases at these positions. The Ik-2 consensus shared five bases with the Ik-1 and Ik-3 heptanucleotide and exhibited great degeneracy outside this sequence. This may permit for the Ik-2 protein to bind with high affinity to a wider range of recognition sequences. Another feature of the oligonucleotides selected by the Ik-3 protein is that 85% of them contained a second consensus (as underlined in Table 2). In contrast, only 50% and 38% of the oligonucleotides selected by Ik-1 and Ik-2 respectively had the potential for a second binding site (as underlined in Table 1 and 3). This may suggest differences in the affinity of Ik-1, Ik-2 and Ik-3 for the selected core motif. Double recognition sequences may allow for an increase in the apparent binding affinity of these proteins for these sites.

Binding site selections were performed as follows. A pool of random oligomers was designed with 25 base pairs of defined sequence at the 5' an 3' (including BamHI and EcoRI restriction sites) and 15 bases of random sequence in the middle. In the first round of selections Ikaros-GST fusions attached to gluathione agarose beads (20µls bead volume) were used in binding assays together with 500,000 cpm of end labeled random primers. After a 20 minute binding reaction on ice the beads were spun down gently and washed twice to three times with ten fold excess of ice cold 1X binding buffer. Bound primers were eluted in 0.1%SDS 10mMTris pH 7.5 recovered radioactivity was determined and then were phenol extracted and precipitated in the presence of 10µgs of glycogen. 1/5th of recovered DNA was reamplified with primers complementary to the defined 5' and 3' sequences with α-P³² dCTP included in the reaction to generate a homogeneously labeled pool of selected oligomers. All probes were gel purified. In higher rounds decreasing amounts of selected oligomers were used in the binding reactions in order to enrich for higher affinity sites (2000,000/100,000 cpm). Five rounds of selections were performed. At the end of the last round of the eluted DNAs were amplified, digested with EcoRI and BamHI restriction enzymes, cloned in PGEM3Z and sequenced with normal and reverse primers. Sequences of selected primers were aligned to a shared motif present in al DNAs.

Fusion protein and DNA binding studies were performed as follows. The coding region of the Ikaros isoforms were PCR amplified with Vent polymerase from their respective cDNAs using primers and cloned into the BamHI/EcoRI sites of pGEXIII. Recombinant plasmids were analyzed by sequencing. Overnight cultures of the appropriate recombinant PGEX vectors were diluted by ten fold and grown at 37° C for 90 minutes before a 3 hours induction with 2mM IPTG at 26°C. Crude bacterial lysates were produced as previously described (Georgopoulos 1992). Ikaros-GST fusions were partially purified on glutathione agarose beads, eluted in buffer D containing 20mMfree glutathione and 0.5 M NaCl at 4°C for 1 hour. Eluted proteins were checked by SDS-Page there concentrations was estimated by the Lowry method and appropriate dilutions were used for DNA binding studies. DNA binding assays were performed. Binding reactions contained 50,000 cpm of labeled oligonucleotides (0.5-lng), 100ngs of the fusion proteins, 0.1µgs of dI/dC and the binding buffer was supplemented with 20µM of ZNCl₂. Binding reactions for methylation interference assays were scaled up ten times and were performed as previously described (Georgopoulos 1990).

### Binding Specificity of Ikaros Isoform Ik-1-5

The binding specificity of the five Ikaros proteins for a single recognition site derived from the selected consensus was tested in a gel retardation assay. A 24 bp oligonucleotide (1K-BS1-T-t-T-T-G-G-G-A-A-T-A-C-Cc) (SEQ ID NO:101) designed to accommodate high affinity binding of the three selecting proteins was tested. The Ik-1 isoform bound this sequence with the highest affinity followed by Ik-2 and Ik-3. The presence of only two or one zinc fingers at the N-terminus of Ik-5 and Ik-4 were not sufficient for their stable interaction with this site. An oligonucleotide containing a second low affinity site in close proximity to the first one was then tested (IK-BS2)TCAGCTTTTGGGAATCTCCTGTCA (SEQ ID NO:150). Four of the isoforms bound to this sequence and only Ik-5 with the single N-terminal finger did not. We then examined the ability of the short Ik-2 core motif to bind to these proteins (IK-BS3, TCAGCTTTTGGGATTCTCCTGTCA (SEQ ID NO:151)). Ik-2 bound equally well to the five versus the seven base pair core, while Ik-1 bound to this site with at least a three fold lower affinity. The Ik-3 and Ik-4 isoforms did not bind to this sequence. Oligonucleotides with non preferred bases and the 3' and 5' flanking positions of the Ik-1/Ik-3 core bound with high affinity the Ik-2 protein, with lower affinity Ik-1 and did not bind Ik-3 (IK-BS5, TCAGCGGGGGGGAATACCCTGTCA (SEQ ID NO:152)). This was in line with the selection data with Ik-2 being the most promiscuous of the three proteins followed by Ik-1 and with Ik-3 being the most restricted in binding specificity.

The Ik-5 protein did not interact with any of these DNAs. However it bound with low affinity but in a sequence specific manner to the δA element of the CD3δ enhancer. The same complex was detected for Ik-4 while Ik-1, Ik-2 and Ik-3 bound this DNA in a qualitative and quantitatively different manner. The weak binding of Ik-4 and Ik-5 to the δA element is probably mediated by the two C-terminal fingers while the high affinity binding of Ik-1, Ik-2 and Ik-3 is mediated by the N-terminal finger domain (also documented by selection sand binding studies with the N-terminal finger domains).

These binding data on the five Ikaros isoforms substantiate further the previously described selections and demonstrate that the Ikaros proteins can bind to overlapping but also distinct recognition sequences.

### Chemical Footprinting of Ikaros Isoforms Ik-1-4 on their cognate sites.

The protein/DNA interactions of Ik-1-4 were further established by chemical footprinting. The IK-BS2 oligonucleotide (TCAGCTTTTGGGAATCTCCTGTCA) (SEQ ID NO:102) that binds with high affinity to the four isoforms was used in a methylation interference assay. On the positive strand all four proteins made similar contacts. The three guanines at positions 2, 3 and 4 of the consensus interfered 100% with the binding of all four proteins. Ik-2 made additional major grove contacts with the guanine at position -5 and with the adenine at position 5. On the negative strand the four portions gave dramatically footprints. Ik-2 made contact only with the guanine at position -3 while Ik-1 made an additional partial contact with position -1. The Ik-3 protein made contacts with position 1 and -2 adenines and -1 and -3 guanines. The Ik-4 protein footprint was the most extensive including the three guanines at position -1, -3 and -4 and the adenine at position -5.

Of the three proteins Ik-3 with the strictest binding specificity made the most DNA contacts. The Ik-2 protein the most promiscuous of the three, made the least DNA contacts. Finally the extensive but qualitative different footprint made by Ik-4 further support the cooperative occupancy of close proximity recognition sites by this protein. These methylation interference data demonstrate that the four Ikaros proteins make qualitative distinct DNA contacts and underling their ability to bind DNA differentially.

### Transcriptional activation by the IK proteins.

We had previously shown that Ik-2 can activate transcription from a multimerized δA element to moderate levels. Here we investigate the ability of four of the Ikaros isoforms to activate transcription from a reiterated copy of a preferred binding site in transient expression assays in NTH-3T3 fibroblasts. The IK-BS3 oligonucleotide which binds Ik-1-4 with high affinity was tested for its ability to stimulate transcription of the tkCAT reporter gene upon expression of these proteins. The activity of this reporter gene detected in this fibroblast cell line was stimulated by 22 fold when cotransfected with an expression vector harboring the Ik-1 cDNA. The activity of this reporter gene was stimulated by the Ik-2 expression vector by 11 fold. However, expression of the Ik-3 and -Ik-4 cDNAs stimulated transcription only by 2 to 3 fold. This suggested that these proteins may function to attenuate transcription from binding sites that also accommodate Ik-1 and Ik-2. (Transient protein expression of the IKaros cDNAs was comparable).

The Ik-1 and Ik-2 enhancer proteins were not able to activate transcription from a mutant binding site which did not bind any of these factors demonstrating that their activation potential is sequence specific.

Since the sequence composition for an Ikaros high affinity binding site is identical to the NFKb motifs present in the IL2-Receptor α and the β-interferon promoters we examined its transcriptional activity in a mature T cell line in the absence and in the presence of mitogenic stimulation. We have chosen the human Jurkat T cell line for the following reasons. First the activity of NFkB recognition sequences that closely match the selected Ikaros binding sites have been extensively studied in this cell line and secondly because we know that the human IKaros gene is highly conserved to the mouse gene in both amino acid composition and splicing variants. In contrast to previous reports we detected high levels of transcriptional activity from this multimerized site which were not further stimulated upon mitogenic treatment. This activity was decreased by five fold when Ikaros antisense expression vectors cotransfected together with this reporter gene. No such effect was detected when reporter genes driven by the RSV or SL3 LTRs were used in a parallel experiment suggesting that transcriptional inhibition by the Ikaros antisense RNA is specific to this site.

Transcriptional activation from a reiterated NFkB variant in NIH3T3 fibroblasts upon expression of Ikaros 1-4 isoforms was determined as follows. The stimulation of CAT activity in the presence of the Ikaros proteins was evaluated as the ratio of activity when cotransfected with a recombinant CDM8/CDM8 vector alone. This data represent an average of three/four experiments with each combination of transfected plasmids per experiment repeated twice. All transfections were normalized to GH levels as described in materials and methods.

Activity and repression of the reiterated NFkB like element in human T cells was determined as follows. The reporter gene under the control of Ik-BS2(FNKB like variant) or of RSV and SL3 LTRs was transfected in Jurat cells in the presence of CDM8 expressing Ikaros antisense plasmids. Fold induction relative to enhancerless plasmid and suppression in presence of antisense RNAs was determined.

Mammalian expression vector and transfection experiments were performed as follows. The five Ikaros isoforms were subcloned into the HindIII-Not I site of the CDM8 expression vector. The tkCAT reporter gene under the control of four sense copies of IKBS 1 IKBS2 IKBS7 and 8 were cotransfected with the appropriate expression vectors and with the SV40 or RSV GH plasmids in the NIH 3T3 fibroblasts and the mature T cell line Jurkat as described previously. Cells were harvested 36-48 hours later and analyzed for CAT activity and Growth hormone levels. Results determined as the average of 3/4 independent experiments where each combination of reporter to expression plasmids was performed twice.

### Target sites for the Ikaros proteins in lymphoid restricted regulatory domains.

Potential high affinity binding sites for the Ikaros proteins were found in the enhancer and promoter regions of the TCR-α -β, and -δ, the CD3 -δ, -ε, and -γ genes the SL3 and HIV LTR and in the regulatory domains of other T cell restricted antigens (Table 5). Some of these sites can bind all four proteins while others interact only with Ik-2 and Ik-1. The selected recognition sequences for the three Ikaros isoforms match closely the NFkB motif present in the promoter of the IL2-R α, in the PRDII element of the β- interferon, and in the H-2K gene. This NFkB site binds with high affinity four of the IKaros proteins.

Related sequences to the Ikaros motif were also found in the above described regulatory domains as well as to the Ikaros motif were also found in the above described regulatory domains as well as in the purine boxes of the IL2 gene in the LYF site of the TDT promoter as well as in the NFkB variant sites of the HIV LTR (Table 5). To investigate the affinity of the Ikaros proteins for these sites we studied their ability to compete with the selected recognition sequences. Base pair substitutions within and outside the seven base pair motif were introduced to match the sequence composition of some of these sites present in the lymphoid and T cell specific regulatory domains. Oligonucleotides with the appropriate base pair changes were used in competition experiments against the consensus motif (IKB-S1).

The Ik-BS2 oligonucleotide, identical to the IL2-Rα NFkB motif, bound to the four proteins with a two fold higher affinity than a single copy of the consensus motif. We believe that this is due to the second low affinity binding site in the opposite strand.

The existence of low affinity binding sites in close proximity in a regulatory domain increases the relative affinity of the Ikaros proteins for these sites. This is clearly the case with δA and possibly with other elements. The occupancy of high affinity binding sites could also be affected by low affinity sites in the immediate region. The apparent binding constant of these proteins for these sites may raise to an even higher value and could dictate the order of target genes activated by the Ikaros enhancers in the developing lymphocyte.

### Cloning of the human Ikaros gene

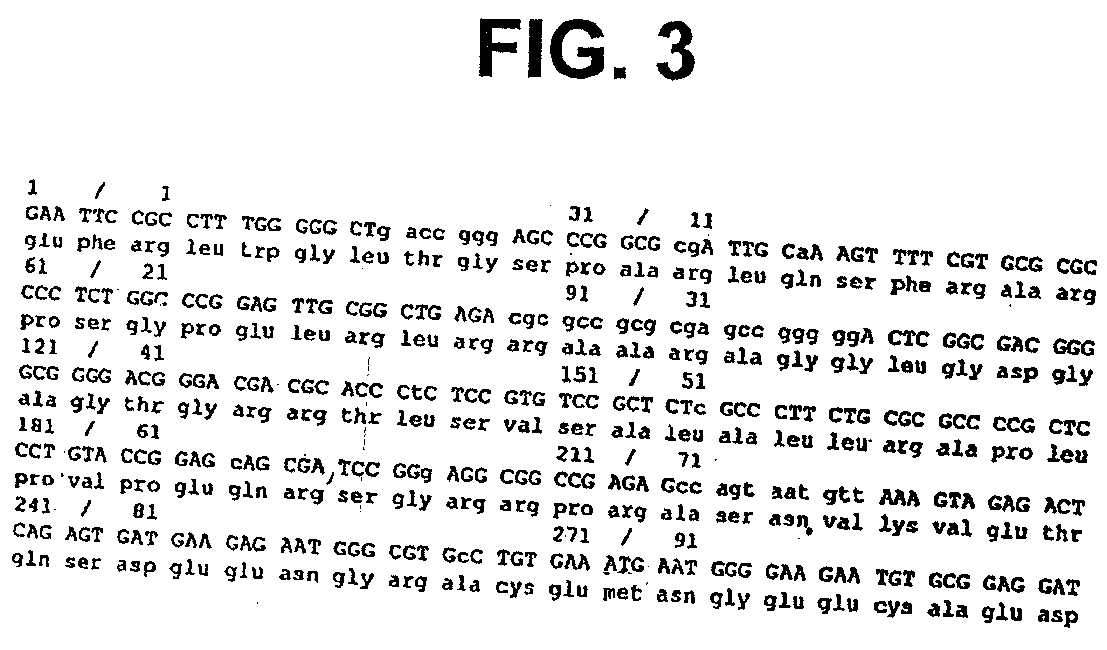

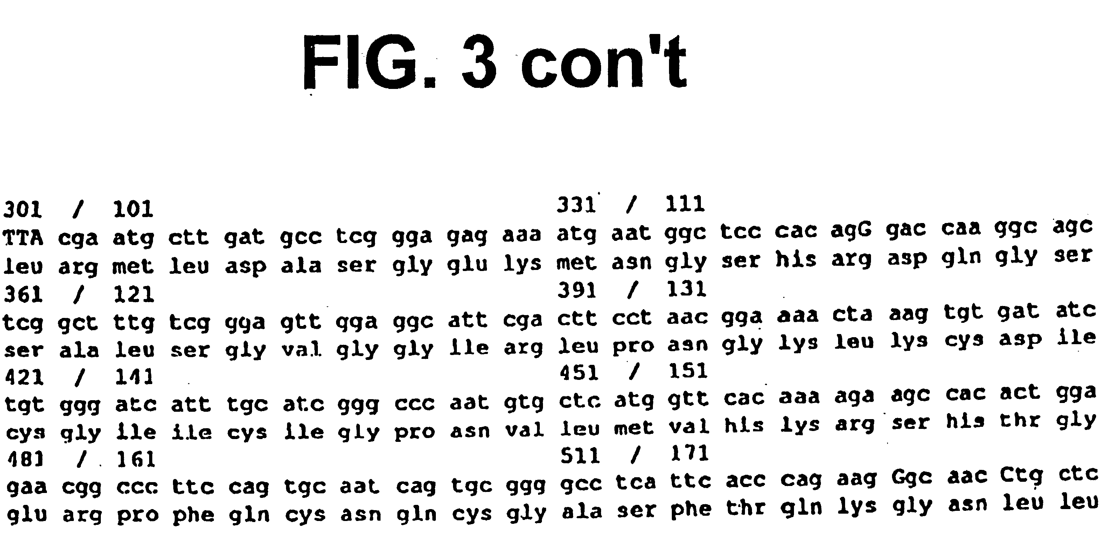

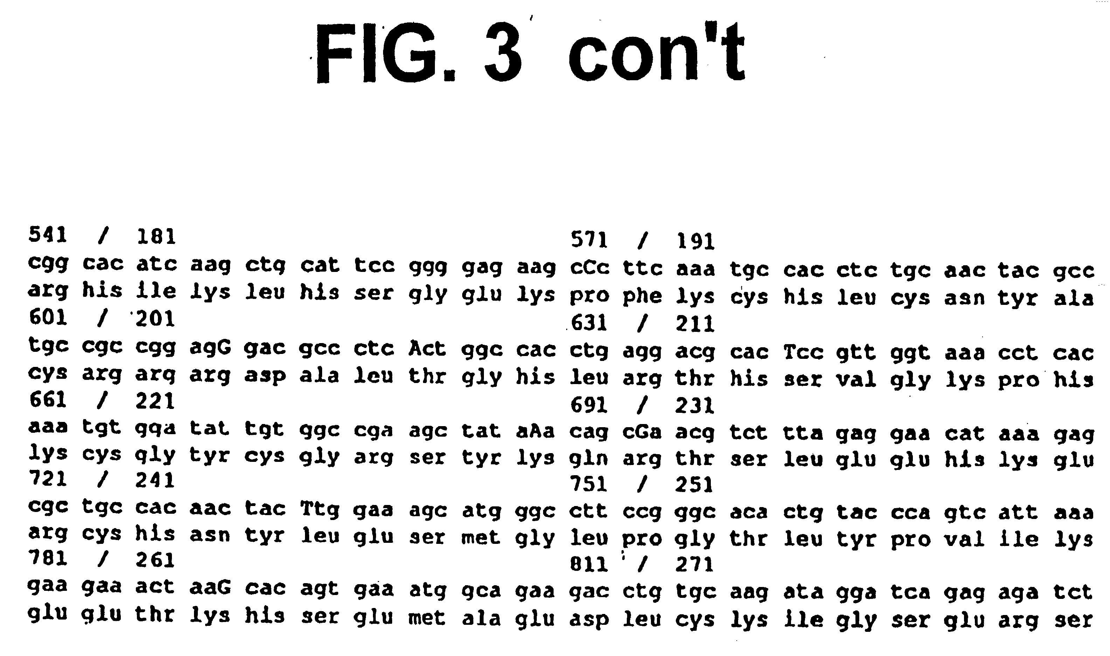

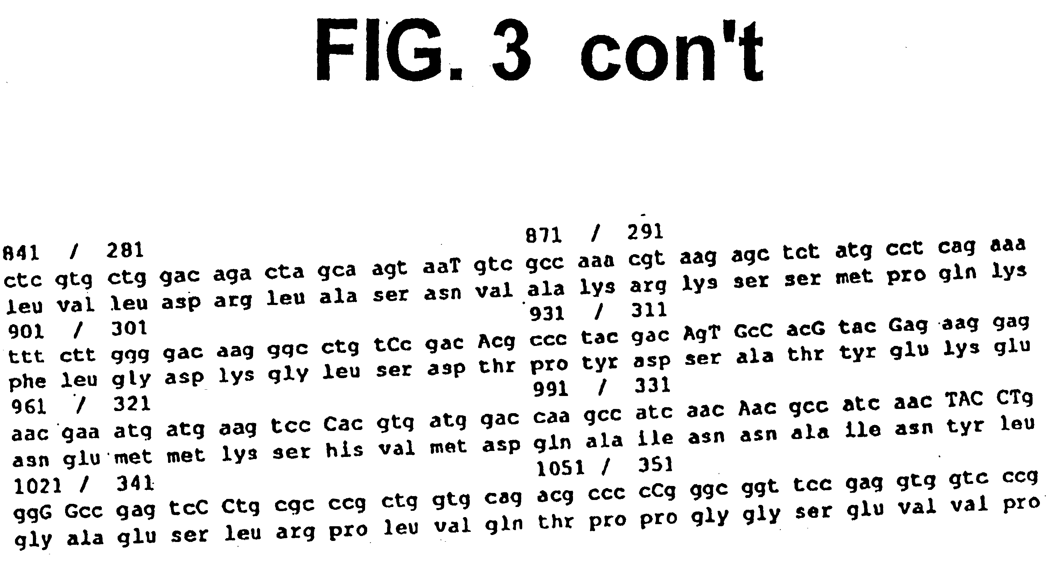

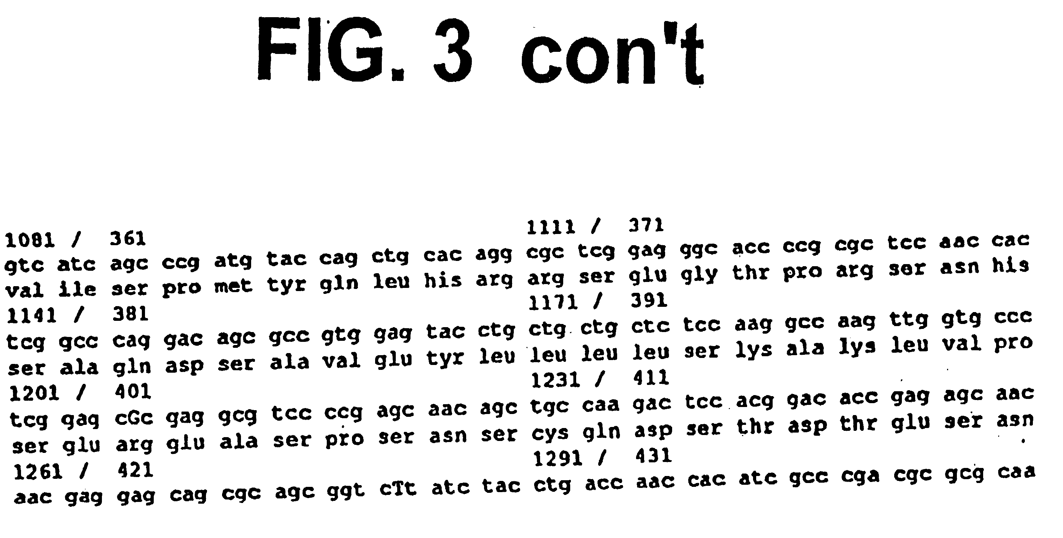

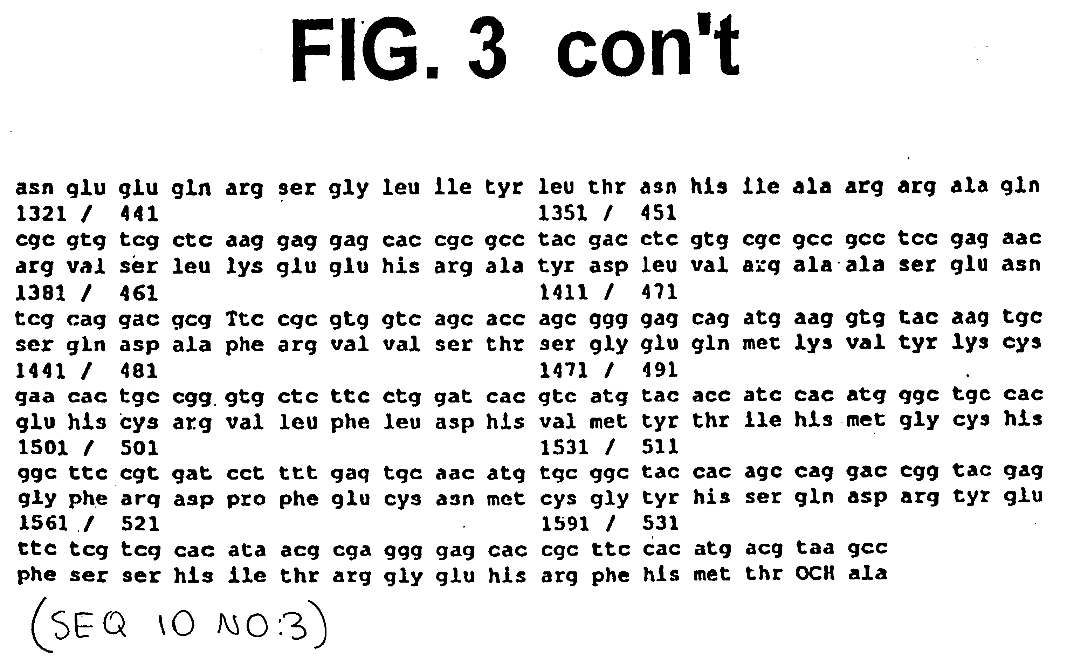

Since the cDNA sequence of the mouse Ikaros gene has been determined the human Ikaros gene can be cloned using any one of a number of techniques known to those skilled in the art. Such cloning techniques are described in detail in (Molecular Cloning: A Laboratory Manual, Sambrook et al. 1989, Second Edition, Cold Spring Harbor Laboratory, Cold Spring Harbor, NY, hereby incorporated by reference). For example, oligonucleotides corresponding to stretches of 1020 amino acid residues of the coding region of mouse Ikaros can be synthesized on an oligonucleotide synthesizer. These oligonucleotides can be used as probes to screen a human cDNA library, e.g., a library from Jurkat T cells, or a human genomic library. Positive clones are sequenced, the sequence is compared to the known amino acid sequence of mouse Ikaros. Functional Ikaros activity encoded by these DNAs can be tested as described herein.
The partial sequence of an Ikaros human cDNA is shown in Fig. 3 (SEQ ID NO:3).

### The Ikaros genomic locus

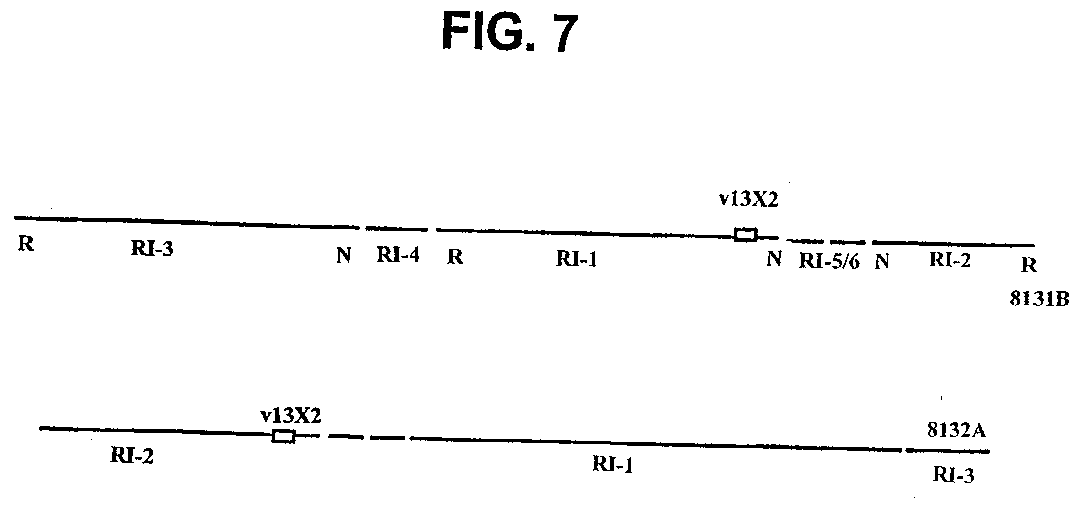

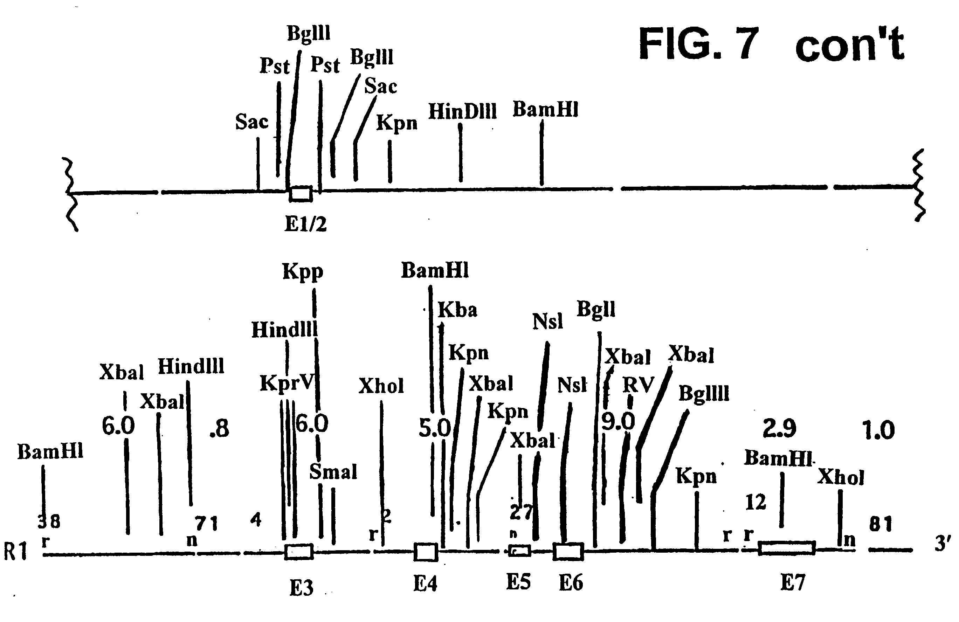

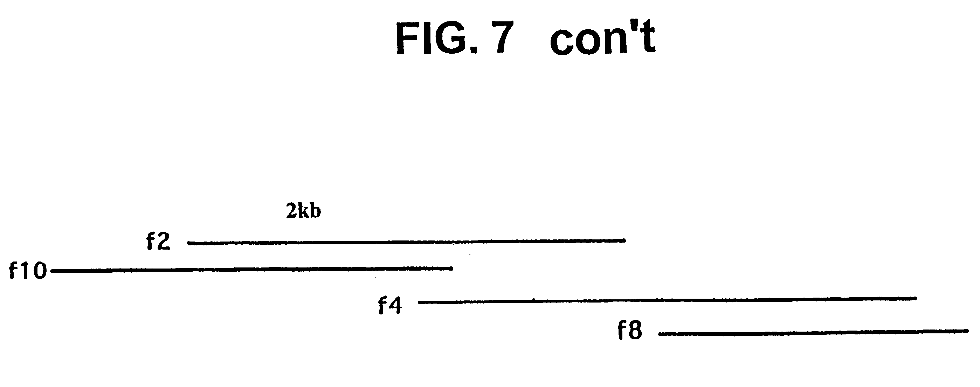

Based on sequence analysis of variant cDNAs, the genomic locus is thought to include about 9-11 exons. Genomic DNAs encompassing most or all of the Ikaros exons present in the genome were isolated by screening a mouse genomic SV129 library made into the λDASH II phage vector using the various Ikaros cDNAs as probes. The Ikaros gene includes at least 80-90kb of genomic sequence which was isolated as distinct but also overlapping genomic clones. Some of the Ikaros genomic clones are indicated in Figs. 7. The exons are depicted as boxes while the introns as lines. The DNA sequence for: the 5' boundary (SEQ ID NO: 143) and the 3' boundary (SEQ ID NO:144) of exon E5; the 5' boundary (SEQ ID NO:145) of exon E3; and the 5' boundary (SEQ ID NO:146) and the 3' boundary (SEQ ID NO:147) of exon E7, were determined.

### Homologous recombination experiments in vitro and in vivo and knockout mice.

To address the role of the lymphoid restricted transcription factor Ikaros in vivo we targeted mutations at the mouse Ikaros genomic locus in embryonic stem cells (E.S). Two targeting vectors carrying distinct deletions at the Ikaros genomic locus were transfected in the J1 E.S line derived from the SV129 mouse (En li, Cell 1992). Homologous recombination events in the E.S cells were scored by a double selection counter selection scheme; G418 and FIAU were used in the media to select for neomycin gene activity and for the absence of thymidine kinase gene activity. The neo gene is located in the middle of the construct while the tk gene is present at the 5' or 3' of the targeting vector and allows for selecting against non-homologous recombination events. E.S. cell lines carrying either mutation one or two were established by Southern analysis and were injected in the blastocysts of Balbe or C57 black mice. The chimeric blastocysts were reimplanted in pseudopregnant mice and gave rise to chimeric animals. Mice which were more than 70% chimeric for the SV129 strain as determined by coat color (agouti vs white or black background) were bred further. Germ line transmission was determined by coat color (agouti) and by Southern analysis of tail DNA. We are in the process of breeding these mice to obtain animals which are homozygous for these mutations.

Both of the targeted mutations are deletions. The first mutation deletes the last exon, E7, which is shared by al the Ikaros isoforms. This should generate proteins which can bind DNA but which cannot activate transcription. These proteins may function as dominant negative regulators of transcription since they can compete for DNA binding with wild type Ikaros proteins but cannot activate transcription. Mice heterozygous for this mutation may exhibit a decrease in the level of expression of genes that rely on the Ikaros proteins for their regulation. These mice may exhibit a less sever phenotype than the ones with total lack of expression of Ikaros proteins. Analysis of these animals may prove to be necessary if the phenotype on mice with total loss of function is severe.

The second mutation (a deletion of exon E3 and E4) should result in a total loss of function of the Ikaros gene. Mice homozygous for this mutation may have a severe impairment of the Ikaros gene. Mice homozygous for this mutation may have a severe impairment of their immune system as a result of altered expression of genes regulated by the Ikaros gene. Possible candidates for Ikaros regulation are TDT (recombination pathway) CD3 complex. TCR complex IL2 gene HIV LTR etc. Lymphoid cell lines derived from these mice can be used to delineate the regulatory pathway that leads to mature T and B cells but the mice themselves can be used to study the complex interaction between the different lineages in the hemopoietic pathway and design in vivo experiments to study and correct immunodeficiency syndromes. Finally, ES cell lines derived from these animals can be studied by in vivo differentiation into the hemopoietic/lymphopoietic lineage.

### Use

The peptides of the invention may be administered to a mammal, particularly a human, in one of the traditional modes (e.g., orally, parenterally, transdermally, or transmucosally), in a sustained release formulation using a biodegradable biocompatible polymer, or by on-site delivery using micelles, gels and liposomes or by transgenic modes.

### Other Embodiments

Nucleic acid encoding all or part of the Ikaros gene can be used to transform cells. For example, the Ikaros gene, e.g., a mis-expressing or mutant form of the Ikaros gene, e.g., a deletion, or DNA encoding an Ikaros protein can be used to transform a cell and to produce a cell in which the cell's genomic Ikaros gene has been replaced by the transformed gene, producing, e.g., a cell deleted for the Ikaros gene. As described above, this approach can be used with cells capable of being grown in culture, e.g., cultured hematopoietic stem cells, to investigate the function of the Ikaros gene.

Analogously, nucleic acid encoding all or part of the Ikaros gene, e.g., a mis-expressing or mutant form of the gene, e.g., a deletion, can be used to transform a cell which subsequently gives rise to a transgenic animal. This approach can be used to create, e.g., a transgenic animal in which the Ikaros gene is, e.g., inactivated, e.g., by a deletion. Homozygous transgenic animals can be made by crosses between the offspring of a founder transgenic animal. Cell or tissue cultures can be derived from a transgenic animal. A subject at risk for a disorder characterized by an abnormality in T cell development or function, e.g., leukemia, can be detected by comparing the structure of the subject's Ikaros gene with the structure of a wild type Ikaros gene. Departure from the wild type structure by, e.g., frameshifts, critical point mutations, deletions, insertions, or translocations, are indicative of risk. The DNA sequence of the coding region of several exons as well as several intron exon boundaries are included herein. Other regions can be obtained or sequenced by methods known to those skilled in the art.

The invention includes any protein which is homologous to an Ikaros protein, e.g., the Ikaros protein shown in SEQ ID NO:2, SEQ ID NO:3, or SEQ ID NO:5, or other isoforms. Also included are: allelic variations; natural mutants; induced mutants, e.g., in vitro deletions; proteins encoded by DNA that hybridizes under high or low (e.g., washing at 2xSSC at 40 C with a probe length of at least 40 nucleotides) stringency conditions to a nucleic acid naturally occurring (for other definitions of high and low stringency see Current Protocols in Molecular Biology, John Wiley & Sons, New York, 1989, 6.3.1 - 6.3.6, hereby incorporated by reference); and polypeptides or proteins specifically bound by antisera to an Ikaros protein, especially by antisera to the active site or binding domain of an Ikaros protein. The term also includes chimeric polypeptides that include an Ikaros protein.

DNA and peptide sequences of the invention can be, e.g., mouse, primate, e.g., human, or non-naturally occurring sequences.

The invention also includes any biologically active fragment or analog of an Ikaros protein. By "biologically active" is meant possessing any in vivo or in vitro activity which is characteristic of an Ikaros isoform, e.g., an isoform shown in (SEQ ID NO:2) or Fig. 3 (SEQ ID NO:3) or (SEQ ID NO:5), e.g., Ikaros activity as described above. Because the Ikaros proteins exhibit a range of physiological properties and because such properties may be attributable to different portions of the Ikaros protein molecule, a useful Ikaros protein fragment or Ikaros protein analog is one which exhibits a biological activity in any one (or more) of a variety of the Ikaros protein assays, for example, the ability to bind to or stimulate transcription from a δA element or an NKFB element, as described above. An Ikaros protein fragment or analog possesses, most preferably 90%, preferably 40%, or at least 10%, of the activity of a naturally occurring Ikaros isoform, e.g., of the Ikaros protein shown in (SEQ ID NO:2), (SEQ ID NO:3) or (SEQ ID NO:5), in any in vivo or in vitro Ikaros assay.

Preferred analogs include Ikaros peptides or exons (or biologically active fragments thereof) whose sequences differ from the wild-type sequence by one or more conservative amino acid substitutions or by one or more non-conservative amino acid substitutions, deletions, or insertions which do not abolish biological activity. Conservative substitutions typically include the substitution of one amino acid for another with similar characteristics, e.g., substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Other conservative substitutions can be taken from the table below.

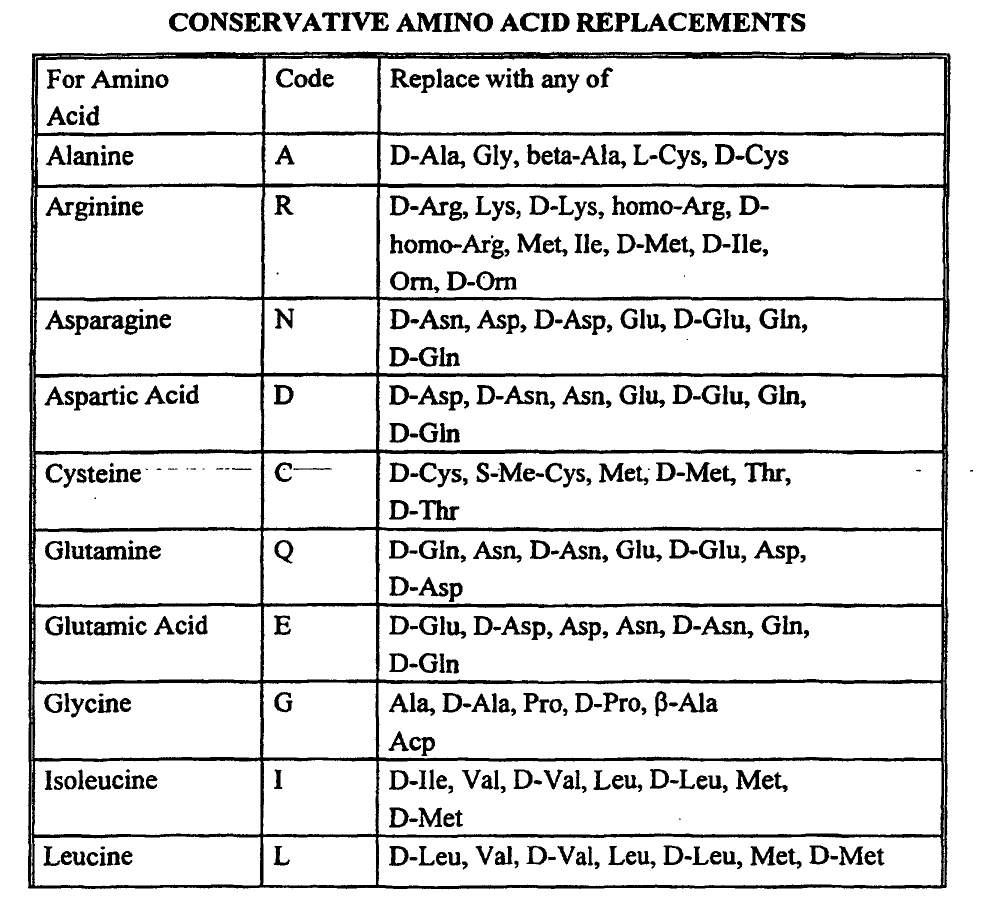

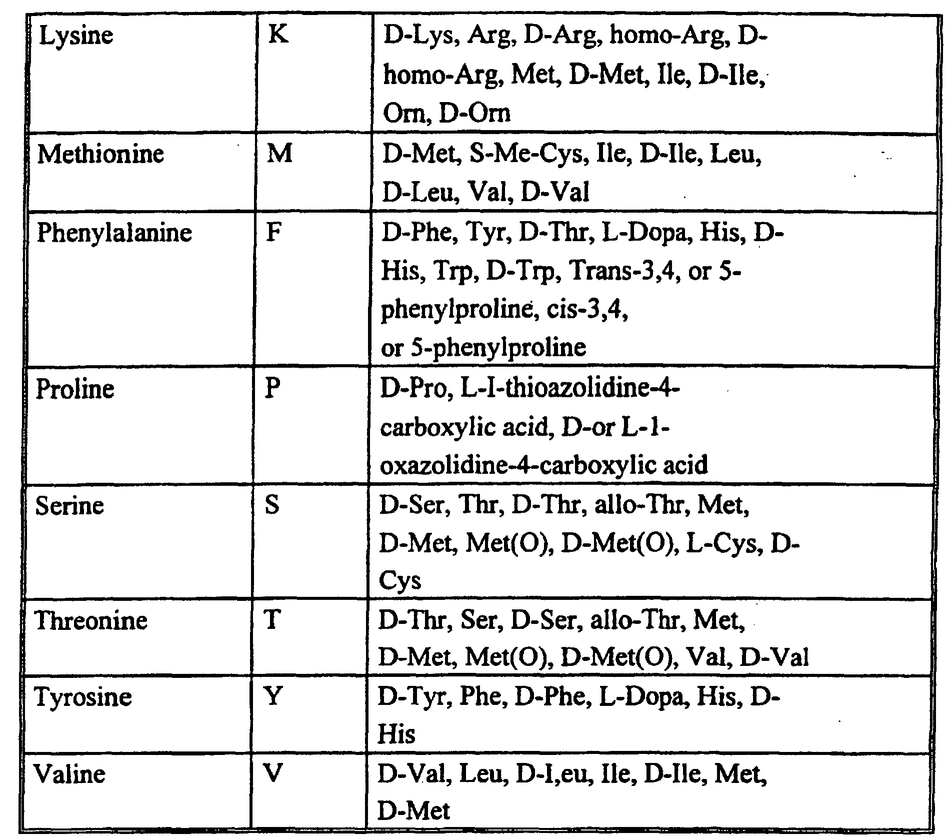

Other useful modifications include those which increase peptide stability; such analogs may contain, for example, one or more non-peptide bonds (which replace peptide bonds) or D-amino acids in the peptide sequence.

Analogs can differ from a naturally occurring Ikaros protein in amino acid sequence or can modified in ways that do not affect sequence, or both. Analogs of the invention will generally exhibit at least 70% more preferably 80%, more preferably 90%, and most preferably 95% or even, 99%, homology with a segment of 20 amino acid residues, preferably more than 40 amino acid residues or more preferably the entire sequence of naturally occurring Ikaros protein sequence.

Alterations in primary sequence include genetic variations, both natural and induced. Also included are analogs that include residues other than naturally occurring L-amino acids, e.g., D-amino acids or non-naturally occurring or synthetic amino acids, e.g.,β or γ amino acids. Alternatively, increased stability may be conferred by cyclizing the peptide molecule.

Nonsequence modification include *in vivo* or *in vitro* chemical derivatization or polypeptides, e.g., acetylation, methylation, phosphorylation, carboxylation, or glycosylation; glycosylation can be modified, e.g., by modifying the glycosylation patterns of a polypeptide during its synthesis and processing or in further processing steps, e.g., by exposing the polypeptide to glycosylation-affecting enzymes derived from cells that normally provide such processing, e.g., mammalian glycosylation enzymes; phosphorylation can be modified by exposing the polypeptide to phosphorylation-altering enzymes, e.g., kinases or phosphatases.

In addition to full-length polypeptides, the invention also includes biologically active fragments of the polypeptides. As used herein, the term "fragment", as applied to a polypeptide, will be of a length described for an Ikaros peptide above and will ordinarily be at least about 20 residues, more typically at least about 40 residues, preferably at least about 60 residues in length.

Fragments of Ikaros peptides or introns can be made by methods known to those skilled in the art, e.g., by expressing Ikaros DNA which has been manipulated in vitro to encode the desired fragment; e.g., by restriction digestion of an Ikaros DNA e.g., the sequence in SEQ ID NO: 1 or SEQ ID NO:2. Analogs can be made by methods known to those skilled in the art, , e.g., by in vitro DNA sequence modifications of the sequence of an Ikaros DNA e.g., the sequence in SEQ ID NO:1 or SEQ ID NO:2. For example, in vitro mutagenesis can be used to convert the DNA sequence of SEQ ID NO: 1 into a sequence which encodes an analog in which one or more amino acid residues has undergone a replacement, e.g., a conservative replacement as described in the table of conservative amino acid substitutions provided herein. Fragments or analogs can be tested by methods known to those skilled in the art for the presence of Ikaros activity.

Also included are Ikaros protein polypeptides containing residues that are not required for biological activity of the peptide, such as residues that are not required for the biological activity of the polypeptide, or that result from alternative mRNA splicing or alternative protein processing events.

The invention also includes nucleic acids encoding the polypeptides of the invention.

In order to obtain an Ikaros protein one can insert Ikaros-encoding DNA into an expression vector, introduce the vector into a cell suitable for expression of the desired protein, and recover and purify the desired protein by prior art methods. Antibodies to Ikaros proteins can be made by immunizing an animal, e.g., a rabbit or mouse, and recovering anti-Ikaros antibodies by prior art methods.

To obtain a specific splicing-product (i.e., a specific isoform) one can make a synthetic structural gene including only the exons which code for the desired splicing product and express the gene as described above.

Other embodiments are within the following claims.

## Claims

1. A DNA sequence encoding a peptide which has an amino acid sequence of at least one Ikaros exon of Figure 4 (SEQ ID NO.5) and which has an Ikaros activity.

2. A DNA sequence which hybridizes under stringent conditions to a DNA sequence according to claim 1.

3. A DNA encoding a peptide comprising a sequence having at least 80% homology to an amino acid sequence of an Ikaros exon of Figure 7 (SEQ ID NO:5) and which has an Ikaros activity.

4. The DNA sequence of claim 3, wherein the sequence has at least 85% homology to an amino acid sequence of an Ikaros exon of Figure 4 (SEQ ID NO:5).

5. The DNA sequence of claim 3, wherein the sequence has at least 90% homology to an amino acid sequence of an Ikaros exon of Figur 4 (SEQ ID NO:5).

6. The DNA sequence of claim 3, wherein the sequence has at least 95% homology to an amino acid sequence of an Ikaros exon of Figure 4 (SEQ ID NO:5).

7. A peptide encoded by a DNA sequence according to any preceding claim.

8. The peptide of claim 7, wherein the exon is selected from the group consisting of exon 1/2, exon 3, exon 4, exon 5, exon 6, and exon 7.

9. A vector comprising a DNA sequence of any of claims 1 to 6.

10. A cultured cell containing the vector of claim 9.

11. A method for manufacture of an Ikaros peptide comprising culturing the cell of claim 10 in a medium to express said Ikaros peptide.

12. A preparation of an antibody directed against an Ikaros peptide of claims 7 or 8.

13. The preparation of claim 12, wherein the antibody is a monoclonal antibody.

14. Use of an Ikaros peptide of claim 7 or 8, a cell selected for the expression of such an Ikaros peptide, or a nucleic acid of any claim 1 to 6 encoding an Ikaros peptide, in the preparation of a composition for the treatment of an animal having an immune disorder.

15. Use of a DNA sequence of claim 1 in the preparation of a diagnostic composition useful for determining if the subject is at risk for an immune disorder by determining if the structure of an Ikaros-gene containing nucleic acid sample from a subject differs from wild-type.

16. A transgenic rodent having a transgene which comprises the DNA sequence of any of claims 1 to 6.

17. Use of an Ikaros peptide of claim 7 or 8, a cell of claim 10 selected for the expression of such an Ikaros peptide, or a nucleic acid of any of claims 1 to 6 encoding an Ikaros peptide, in the preparation of a composition for the treatment of an animal having a disorder of the corpus striatum.

18. Use of a DNA sequence of claim 1 in the preparation of a diagnostic composition useful for determining if the subject is at risk for a disorder of the corpus striatum by determining if the structure of an Ikaros-gene containing nucleic acid sample from a subject differs from wild-type.

## Patentansprüche

1. Eine DNS-Sequenz, die für ein Peptid codiert, welches eine Aminosäuresequenz von mindestens einem Ikaros-Exon der Abbildung 4 (SEQ ID No: 5) hat und welches eine Ikaros-Aktivität hat.

2. Eine DNS-Sequenz, welche unter stringenten Bedingungen mit einer DNS-Sequenz nach Anspruch 1 hybridisiert.

3. Eine DNS, welche für ein Peptid codiert, umfassend eine Sequenz mit mindestens 80% Homologie zu einer Aminosäuresequenz von einem Ikaros-Exon der Abbildung 4 (SEQ ID No: 5) und welches eine Ikaros-Aktivität besitzt.

4. DNS-Sequenz nach Anspruch 3, worin die Sequenz mindestens 85% Homologie zu einer Aminosäuresequenz von einem Ikaros-Exon der Abbildung 4 (SEQ ID No: 5) hat.

5. DNS-Sequenz nach Anspruch 3, worin die Sequenz mindestens 90% Homologie zu einer Aminosäuresequenz von einem Ikaros-Exon der Abbildung 4 (SEQ ID No: 5) hat.

6. DNS-Sequenz nach Anspruch 3, worin die Sequenz mindestens 95% Homologie zu einer Aminosäuresequenz von einem Ikaros-Exon der Abbildung 4 (SEQ ID No:5) hat.

7. Ein Peptid, welches durch eine DNS-Sequenz nach einem beliebigen vorangehenden Anspruch codiert wird.

8. Peptid nach Anspruch 7, worin das Exon ausgewählt ist aus einer Gruppe bestehend aus Exon 1/2, Exon 3, Exon 4, Exon 5, Exon 6 und Exon 7.

9. Vektor umfassend eine DNS-Sequenz nach einem beliebigen der Ansprüche 1 bis 6.

10. Kultivierte Zelle, welche den Vektor nach Anspruch 9 beinhaltet.

11. Verfahren zur Herstellung eines Ikaros-Peptids umfassend das Kultivieren der Zelle nach Anspruch 10 in einem Medium zur Expression des Ikaros-Peptids.

12. Zubereitung eines Antikörpers gerichtet gegen ein Ikaros-Peptid nach Anspruch 7 oder 8.

13. Zubereitung nach Anspruch 12, worin der Antikörper ein monoklonaler Antikörper ist.

14. Verwendung eines Ikaros-Peptids nach Anspruch 7 oder 8, einer Zelle ausgewählt zur Expression eines solchen Ikaros-Peptids, oder einer Nukleinsäure eines beliebigen der Ansprüche 1 bis 6, welche für ein Ikaros-Peptid codiert, in der Zubereitung einer Zusammensetzung zur Behandlung eines Tieres mit einer Immunstörung.

15. Verwendung einer DNS-Sequenz nach Anspruch 1 in der Zubereitung einer diagnostischen Zusammensetzung zur Bestimmung, ob bei dem Individuum das Risiko für eine Immunstörung besteht, durch Bestimmung, ob die Struktur einer Nukleinsäure-Probe eines Individuums, welche ein Ikaros-Gen enthält, sich vom Wildtyp unterscheidet.

16. Transgener Nager mit einem Transgen, welches die DNS-Sequenz nach einem beliebigen der Ansprüche 1 bis 6 umfasst.

17. Verwendung eines Ikaros-Peptids nach Anspruch 7 oder 8, einer Zelle nach Anspruch 10 ausgewählt zur Expression eines solchen Ikaros-Peptids, oder einer Nukleinsäure nach einem beliebigen der Ansprüche 1 bis 6, welche für ein Ikaros-Peptid codiert, in der Zubereitung einer Zusammensetzung zur Behandlung eines Tieres mit einer Störung des Corpus striatum.

18. Verwendung einer DNS-Sequenz nach Anspruch 1 in der Zubereitung einer diagnostischen Zusammensetzung anwendbar zur Bestimmung, ob bei dem Individuum das Risiko für eine Störung des Corpus striatum besteht durch Bestimmung, ob die Struktur einer Nukleinsäure-Probe eines Individuums, welche ein Ikaros-Gen enthält, sich vom Wildtyp unterscheidet.

## Revendications

1. Séquence d'ADN codant un peptide qui a une séquence d'acides aminés d'au moins un exon d'Ikaros de la figure 4 (SEQ ID NO: 5) et qui a l'activité d'un Ikaros.

2. Séquence d'ADN qui s'hybride dans des conditions rigoureuses à une séquence d'ADN selon la revendication 1.

3. ADN codant un peptide comprenant une séquence ayant au moins 80% d'homologie avec une séquence d'acides aminés d'un exon d'Ikaros de la figure 4 (SEQ ID NO: 5) et qui a l'activité d'un Ikaros.

4. Séquence d'ADN de la revendication 3, la séquence ayant au moins 85% d'homologie avec une séquence d'acides aminés d'un exon d'Ikaros de la figure 4 (SEQ ID NO: 5).

5. Séquence d'ADN de la revendication 3, la séquence ayant au moins 90% d'homologie avec une séquence d'acides aminés d'un exon d'Ikaros de la figure 4 (SEQ ID NO: 5).

6. Séquence d'ADN de la revendication 3, la séquence ayant au moins 95% d'homologie avec une séquence d'acides aminés d'un exon d'Ikaros de la figure 4 (SEQ ID NO: 5).

7. Peptide codé par une séquence d'ADN selon l'une quelconque des revendications précédentes.

8. Peptide de la revendication 7, dans lequel l'exon est choisi dans le groupe constitué par l'exon 1/2, l'exon 3, l'exon 4, l'exon 5, l'exon 6 et l'exon 7.

9. Vecteur comprenant une séquence d'ADN de l'une quelconque des revendications 1 à 6.

10. Cellule cultivée contenant le vecteur de la revendication 9.

11. Procédé pour la fabrication d'un peptide Ikaros, comprenant la culture de la cellule de la revendication 10 dans un milieu pour exprimer ledit peptide Ikaros.

12. Préparation d'un anticorps dirigé contre un peptide Ikaros des revendications 7 ou 8.

13. Préparation de la revendication 12, dans laquelle l'anticorps est un anticorps monoclonal.

14. Utilisation d'un peptide Ikaros de la revendication 7 ou 8, d'une cellule choisie pour l'expression d'un tel peptide Ikaros, ou d'un acide nucléique de l'une quelconque des revendications 1 à 6 codant un peptide Ikaros, dans la préparation d'une composition pour le traitement d'un animal ayant un trouble immunitaire.

15. Utilisation d'une séquence d'ADN de la revendication 1 dans la préparation d'une composition diagnostique utile pour déterminer si le sujet présente un risque de trouble immunitaire, en déterminant si la structure d'un échantillon d'acide nucléique contenant un gène Ikaros d'un sujet diffère du type sauvage.

16. Rongeur transgénique ayant un transgène qui comprend la séquence d'ADN de l'une quelconque des revendications 1 à 6.

17. Utilisation d'un peptide Ikaros de la revendication 7 ou 8, d'une cellule de la revendication 10 choisie pour l'expression d'un tel peptide Ikaros, ou d'un acide nucléique de l'une quelconque des revendications 1 à 6 codant un peptide Ikaros, dans la préparation d'une composition pour le traitement d'un animal ayant une affection du corps strié.

18. Utilisation d'une séquence d'ADN de la revendication 1 dans la préparation d'une composition diagnostique utile pour déterminer si le sujet présente un risque d'affection du corps strié, en déterminant si la structure d'un échantillon d'acide nucléique contenant un gène Ikaros d'un sujet diffère du type sauvage.
